# FASCICULE DE BREVET EUROPEEN

(11) **EP 1 274 390 B1**
(45) Date de publication et mention de la délivrance du brevet: **21.12.2005**
(21) Numéro de dépôt: 01919536.1
(22) Date de dépôt: 21.03.2001
(51) Int. Cl.: A61K 7/13

(54) **COMPOSITION POUR LA TEINTURE D'OXYDATION DES FIBRES KERATINIQUES COMPRENANT UN DERIVE DE LA 3,5-DIAMINO-PYRIDINE ET UN POLYMERE CATIONIQUE OU AMPHOTERE**
ZUSAMMENSETZUNG FÜR DIE OXIDATIONSFÄRBUNG DER KERATINISCHEN FASERN, DIE EIN 3,5-DIAMINOPYRIDINDERIVAT UND EIN KATIONISCHES ODER AMPHOTERISCHES POLYMER ENTHÄLT
OXIDATION DYEING COMPOSITION FOR KERATINOUS FIBRES COMPRISING A 3,5-DIAMINO-PYRIDINE DERIVATIVE AND A CATIONIC OR AMPHOTERIC POLYMER

(30) Priorité: 12.04.2000 FR 0004720
(43) Date de publication de la demande: 15.01.2003
(73) Titulaire: L'OREAL, 75008 Paris (FR)
(72) Inventeur: AUDOUSSET, Marie-Pascale, F-92600 Asnières (FR)
(74) Mandataire: Wattremez, Catherine
(86) Numéro de dépôt international: PCT/FR2001/000847
(87) Numéro de publication internationale: WO 2001/078669

(56) Documents cités:
- DE-A- 4 018 335
- US-A- 4 698 065
- US-A- 4 923 977
- US-A- 5 279 616
- US-A- 5 743 919

## Description

La présente invention concerne une composition pour la teinture d'oxydation des fibres kératiniques, en particulier des fibres kératiniques humaines telles que les cheveux, comprenant, dans un milieu approprié pour la teinture, au moins un coupleur choisi parmi les dérivés de la 3,5-diamino-pyridine et leurs sels d'addition avec un acide, au moins un précurseur de colorant d'oxydation, et au moins un polymère cationique ou amphotère particulier défini ci-après.

II est connu de teindre les fibres kératiniques et en particulier les cheveux humains, avec des compositions de teinture contenant dés précurseurs de colorants d'oxydation, généralement appelés "bases d'oxydation", en particulier des ortho- ou para- phénylènediamines, des ortho- ou para- aminophénois, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Ainsi, il a déjà été proposé, notamment dans les brevets US-4473375 ou DE-3132885 des compositions de teinture d'oxydation contenant certains dérivés de la 3,5-diamino-pyridine à titre de coupleur, en association avec des bases d'oxydation classiquement utilisées en teinture d'oxydation.
Cependant, les colorations obtenues en mettant en oeuvre ces compositions ne sont pas toujours assez puissantes, chromatiques, ou résistantes aux différentes agressions que peuvent subir les cheveux.

Le document brevet EP-A-1 138 318, opposable uniquement au titre de l'Article 54(3) CBE, décrit aux exemples 1-3 et 5 une composition pour la teinture d'oxydation des fibres keratiniques, en particulier des fibres kératiniques humaines telles que les cheveux, comprenant, dans un milieu approprié pour la teinture, (a) à titre de coupleur, au moins un dérivé de 3, 5-diamino-pyridine et, (b) au moins un précurseur de colorant d'oxydation, caractérisée par le fait qu'elle comprend en outre un polymère cationique dérivé de guar.

Or, la Demanderesse vient maintenant de découvrir qu'il est possible d'obtenir de façon totalement inattendue et surprenante, de nouvelles teintures d'oxydation, capables de conduire à des colorations aux nuances variées, chromatiques, puissantes, esthétiques, peu sélectives, et résistant bien aux diverses agressions que peuvent subir les fibres, en associant au moins un coupleur choisi parmi les 3,5-diamino-pyridines de formule (I) définie ci-après et leurs sels d'addition avec un acide, au moins un précurseur de colorant d'oxydation, et au moins un polymère cationique ou amphotère particulier défini ci-après.

Cette découverte est à la base de la présente invention.

L'invention a donc pour premier objet une composition pour la teinture d'oxydation des fibres kératiniques et en particulier des fibres kératiniques humaines telles que les cheveux, comprenant dans un milieu approprié pour la teinture,
**(a) à titre de coupleur,** au moins un dérivé de 3,5-diamino-pyridine de formule (I) suivante : 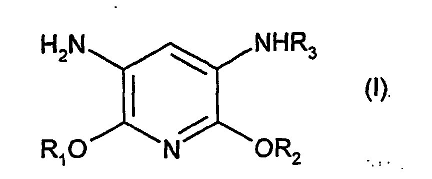 dans laquelle :
   - R₁ et R₂, identiques ou différents, représentent un radical alkyle en C₁-C₄, monohydroxyalkyle en C₁-C₄, ou polyhydroxyalkyle en C₂-C₄,
   - R₃ représente un atome d'hydrogène, un radical alkyle en C₁-C₄, monohydroxyalkyle en C₁-C₄ ou polyhydroxyalkyle en C₂-C₄
   ou l'un de ses sels d'addition avec un acide ;
   et,
**(b)** au moins un précurseur de colorant d'oxydation,
**caractérisée** par le fait qu'elle comprend en outre au moins un polymère cationique ou amphotère choisi dans le groupe formé par :
**(i)** les homopolymères et les copolymères d'halogénure de méthacryloyloxyéthyltriméthylammonium ;
**(ii)** les polyammonium quaternaire comportant des motifs récurrents de formules (I) ou (II) suivantes : 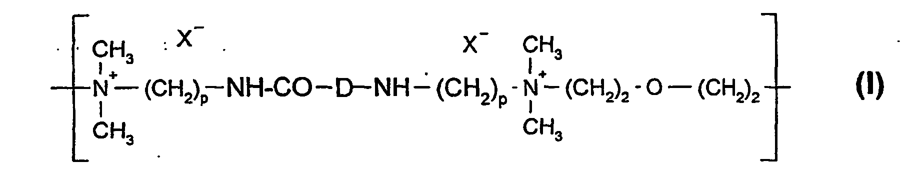 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ; 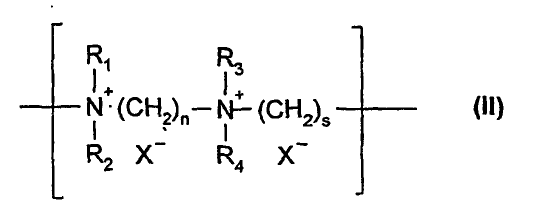 dans laquelle R₁, R₂, R₃ et R₄, identiques ou différents, désignent un radical alkyle ou hydroxyalkyle ayant de 1 à 4 atomes de carbone environ, n et s sont des nombres entiers variant de 2 à 20 et X⁻ est un anion ;
**(iii)** les copolymères de vinylpyrrolidone à motifs cationiques ;
**(iv)** les silicones aminées ;
**(v)** les homopolymères d'halogénure de diméthyldiallylammonium et les copolymères d'halogénure de diméthyldiallylammonium et d'acrylamide;
**(vi)** les copolymères d'halogénure de diméthyldiallylammonium et d'acide acrylique ;
**(vii)** les copolymères d'acide (méth)acrylique, ou d'acide maléique, ou d'acide alpha-chloracrylique et de dialkylaminoalkyl(méth)acrylate ou de dialkylaminoalkyl(méth)acrylamide ;
**(viii)** le copolymère acrylate de sodium et de chlorure d'acrylamidopropyltriméthylammonium ;
**(ix)** l'Octylacrylamide/Acrylates/Butylaminoethyl Methacrylate Copolymer ;
**(x)** les polymères comportant des motifs zwittérioniques de formule (III) suivante : 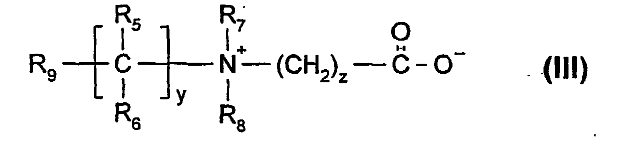 dans laquelle R₉ désigne un groupement acrylate, méthacrylate, acrylamide ou méthacrylamide, y et z représentent un nombre entier de 1 à 3, R₅ et R₆représentent un atome d'hydrogène, méthyle, éthyle ou propyle, R₇ et R₈ représentent un atome d'hydrogène ou un radical alkyle de telle façon que la somme des atomes de carbone dans R₇ et R₈ ne dépasse pas 10 ;
**(xii)** Les polymères répondant à la formule générale (VII) : 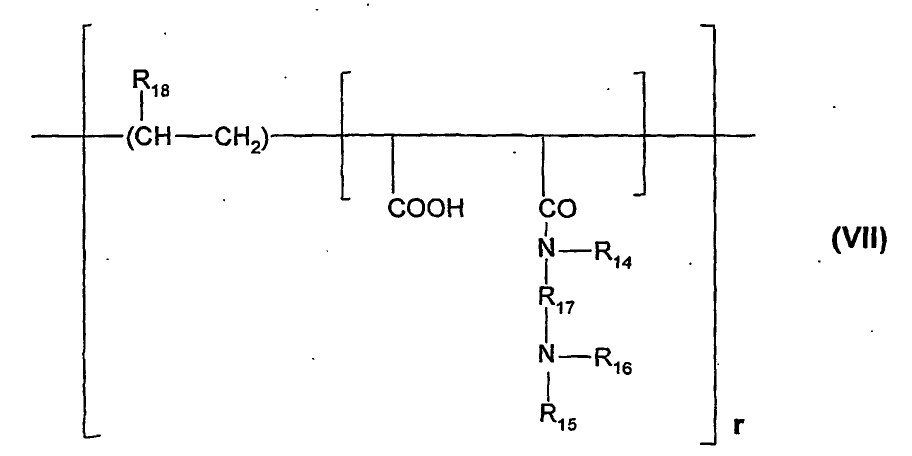 dans laquelle R₁₈ représente un atome d'hydrogène, un radical CH₃O, CH₃CH₂O, phényle, R₁₄ désigne l'hydrogène ou un radical alkyle(C₁-C₆), R₁₅ désigne l'hydrogène ou un radical alkyle(C₁-C₆), R₁₆ désigne un radical alkyle(C₁-C₆), ou un radical répondant à la formule : -R₁₇-N(R₁₅)₂, R₁₇ représentant un groupement -CH₂-CH₂- , -CH₂-CH₂-CH₂- , -CH₂-CH(CH₃)- , et R₁₅ ayant les significations mentionnées ci-dessus ;
**(xiii)** les copolymères alkyl(C₁-C₅)vinyléther / anhydride maléique modifié partiellement par semiamidification avec une N,N-dialkylaminoalkylamine ou par semiestérification avec une N,N-dialcanolamine.

La composition tinctoriale conforme à l'invention ainsi définie, conduit après mélange avec une composition oxydante, à des colorations dans des nuances variées, chromatiques, puissantes, esthétiques, présentant une faible sélectivité et d'excellentes propriétés de résistances à la fois vis-à-vis des agents atmosphériques tels que la lumière et les intempéries et vis-à-vis de la transpiration et des différents traitements que peuvent subir les cheveux.

Un autre objet de l'invention porte sur une composition prête à l'emploi pour la teinture d'oxydation des fibres kératiniques qui comprend, dans un milieu approprié pour la teinture, au moins une 3,5-diamino-pyridine de formule (I), au moins un précurseur de colorant d'oxydation, au moins un polymère cationique ou amphotère ci-dessus défini et au moins un agent oxydant.

Par composition prête à l'emploi, on entend au sens de la présente invention, toute composition destinée à être appliquée immédiatement sur les fibres kératiniques.

L'invention vise également un procédé de teinture d'oxydation des fibres kératiniques, et en particulier des fibres kératiniques humaines telles que les cheveux, consistant à appliquer sur les fibres une composition colorante contenant dans un milieu approprié pour la teinture, au moins une 3,5-diamino-pyridine de formule (I) et au moins un précurseur de colorant d'oxydation, la couleur étant révélée à pH alcalin, neutre ou acide à l'aide d'une composition oxydante qui est mélangée juste au moment de l'emploi à la composition colorante ou qui est appliquée séquentiellement sans rinçage intermédiaire, au moins un polymère cationique ou amphotère défini selon l'invention étant présent dans la composition colorante et/ou oxydante.

L'invention a également pour objet des dispositifs de teinture à plusieurs compartiments ou "kits" pour la teinture d'oxydation des fibres kératiniques, en particulier des fibres kératiniques humaines telles que les cheveux.
De tels dispositifs comportent un premier compartiment contenant au moins une 3,5-diamino-pyridine de formule (I) et au moins un précurseur de colorant d'oxydation et un deuxième compartiment contenant un agent oxydant, au moins un polymère cationique ou amphotère défini selon l'invention, étant présent dans le premier compartiment et/ou dans te second compartiment.

Un autre dispositif de teinture à plusieurs compartiments comporte au moins un compartiment contenant au moins une 3,5-diamino-pyridine de formule (I) et au moins un précurseur de colorant d'oxydation, au moins un compartiment contenant au moins un polymère cationique ou amphotère défini selon l'invention, et au moins un autre compartiment contenant au moins un agent oxydant.

Mais d'autres caractéristiques, aspects, objets et avantages de l'invention apparaîtront encore plus clairement à la lecture de la description et des exemples qui suivent.

Parmi les dérivés de 3,5-diaminopyridine de formule (I) conformes à l'invention, on peut citer la 2,6-diméthoxy-3,5-diamino-pyridine, la 2,6-diéthoxy-3,5-diamino-pyridine, la 2,6-di- (β-hydroxyéthyloxy)-3,5-diamino-pyridine et leurs sels d'addition avec un acide.

Selon l'invention, la composition tinctoriale renferme de préférence la 2,6-diméthoxy-3,5-diamino-pyridine, ou au moins l'un de ses sels d'addition avec un acide.

Le ou les dérivés de 3,5-diamino-pyridine de formule (I) utilisables dans la composition tinctoriale conforme à l'invention représentent de préférence de 0,0001 à 10 % en poids environ du poids total de la composition tinctoriale, et encore plus préférentiellement de 0,005 à 5 % en poids environ de ce poids.

La composition tinctoriale conforme à l'invention renferme au moins un précurseur de colorant d'oxydation ou base d'oxydation.
La nature de ces bases d'oxydation n'est pas critique.
Elles peuvent notamment être choisies parmi les paraphénylènediamines, les bases doubles, les para-aminophénols, les ortho-aminophénols et les bases d'oxydation hétérocycliques et leurs sels d'addition avec un acide.

Parmi les paraphénylènediamines utilisables à titre de base d'oxydation dans les composition tinctoriales conformes à l'invention, on peut notamment citer les composés de formule (II) suivante et leurs sels d'addition avec un acide : 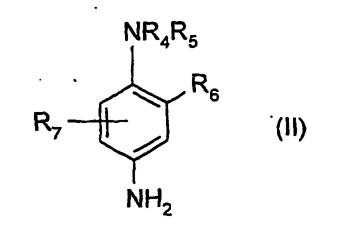 dans laquelle :
- R₄ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
- R₅ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄) ou alkyle en C₁-C₄ substitué par un groupement azoté ;
- R₆ représente un atome d'hydrogène, un atome d'halogène tel qu'un atome de chlore, de brome, d'iode ou de fluor, un radical alkyle en C₁-C₄, monohydroxyalkyle en C₁-C₄, hydroxyalcoxy en C₁-C₄, acétylaminoalcoxy en C₁-C₄, mésylaminoalcoxy en C₁-C₄ ou carbamoylaminoalcoxy en C₁-C₄,
- R₇ représente un atome d'hydrogène, d'halogène ou un radical alkyle en C₁-C₄.

Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (II) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl - 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediàmine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, 2-méthyl-1-N-β-hydroxyéthyl paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.
Parmi les bases doubles utilisables à titre de bases d'oxydation dans les compositions tinctoriales conformes à l'invention, on peut notamment citer les composés répondant à la formule (III) suivante, et leurs sels d'addition avec un acide : 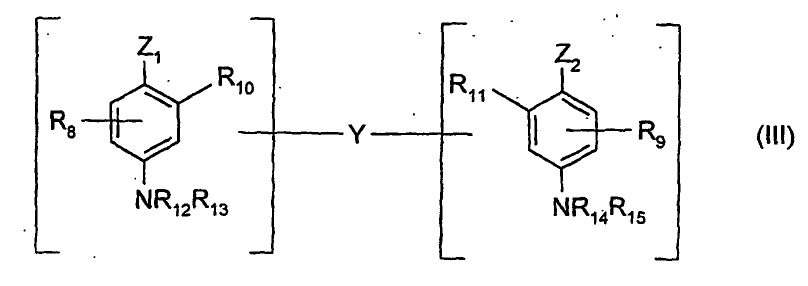 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₈ et R₉ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₁₀, R₁₁, R₁₂, R₁₃, R₁₄ et R₁₅, identiques ou différents, représentent un atome d'hydrogène, un bras de liaison Y ou un radical alkyle en C₁-C₄ ;
étant entendu que les composés de formule (III) ne comportent qu'un seul bras de liaison Y par molécule.

Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés répondant à la formule (IV) suivante, et leurs sels d'addition avec un acide : 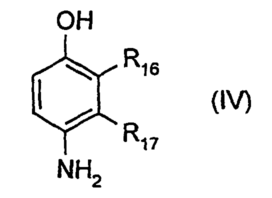 dans laquelle :
- R₁₆ représente un atome d'hydrogène ou d'halogène, un radical alkyle en C₁-C₄, monohydroxyalkyle en C₁-C₄, alcoxy(C₁-C₄)alkyle(C₁-C₄), aminoalkyle en C₁-C₄ ou hydroxyalkyl(C₁-C₄)aminoalkyle en C₁-C₄,
- R₁₇ représente un atome d'hydrogène ou d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₁₆ ou R₁₇ représente un atome d'hydrogène.

Parmi les para-aminophénols de fomnule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)-amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine, leurs formes tautomères, lorsqu'il existe un équilibre tautomérique et leurs sels d'addition avec un acide.

Parmi les dérivés pyrazoliques, on peut plus particulièrement citer les composés décrits dans les brevets DE-3 843 892, DE-4 133 957 et demandes de brevet WO-94/08969, WO-94/08970, FR-A-2 733 749 et DE-195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Selon une forme de réalisation préférée de l'invention la ou les bases d'oxydation sont choisies parmi les paraphénylènediamines.

La ou les bases d'oxydation représentent de préférence de 0,0005 à 12 % en poids environ du poids total de la composition tinctoriale conforme à l'invention, et encore plus préférentiellement de 0,005 à 8 % en poids environ de ce poids:

### Polymères cationiques et amphotères selon l'invention.

**(i)** Parmi les polymères d'halogénure de méthacryloyloxyéthyltriméthylammonium utilisables selon l'invention, on peut citer en particulier les produits qui sont dénommés dans le dictionnaire CTFA (5ème édition, 1993) "Polyquaternium 37" , "Polyquaternium 32" et "Polyquaternium 35" , qui correspondent respectivement, en ce qui concerne le "Polyquaternium 37", au poly(chlorure de méthacryloyloxyéthyltriméthyl-ammonium) réticulé, en dispersion à 50% dans de l'huile minérale, et vendu sous la dénomination Salcare SC95 par la société Allied Colloids, en ce qui concerne le "Polyquaternium 32", au copolymère réticulé de l'acrylamide et du chlorure de méthacryloyloxyéthyltriméthylammonium (20/80 en poids), en dispersion à 50% dans de l'huile minérale, et vendu sous la dénomination Salcare SC92 par la société Allied Colloids, et en ce qui concerne le "Polyquaternium 35", au méthosulfate du copolymère de méthacryloyloxyéthyltriméthylammonium et de méthacryloyloxyéthyldiméthylacétylammonium, vendu sous la dénomination Plex 7525L par la société Rohm GmbH.
**(ii)** Les polyammonium quaternaire de formule (I) utilisables selon l'invention peuvent être préparés selon les procédés décrits dans les brevets U.S.A. n° 4 157 388, 4 390 689, 4 702 906, 4 719 282. Ils sont notamment décrits dans la demande de brevet EP-A-122 324.
   Parmi ces polymères, on préfère ceux de masse moléculaire mesurée par RMN du Carbone 13 inférieure à 100000, pour lesquels, p est égal à 3, et,
   a) D représente un groupement -(CH₂)₄-CO-, X désigne un atome de chlore, la masse moléculaire mesurée par RMN du Carbone 13 (RMN¹³C) étant d'environ 5600 ; un polymère de ce type est proposé par la société MIRANOL sous le nom de MIRAPOL-AD1,
   b) D représente un groupement -(CH₂)₇-CO-, X désigne un atome de chlore, la masse moléculaire mesurée par RMN du Carbone 13 (RMN¹³C) étant d'environ 8100 ; un polymère de ce type est proposé par la société MIRANOL sous le nom de MIRAPOL-AZ1,
   c) D désigne la valeur zéro, X désigne un atome de chlore, la masse moléculaire mesurée par RMN du Carbone 13, (RMN¹³C) étant d'environ 25500 ; un polymère de ce type est vendu par la société MIRANOL sous le nom MIRAPOL-A15,
   d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).

   Plus particulièrement encore, on préfère selon l'invention le polymère à motifs de formule (1) dans laquelle p est égal à 3, D désigne la valeur zéro, X désigne un atome de chlore, la masse moléculaire mesurée par RMN du Carbone 13, (RMN¹³C) étant d'environ 25500.
   Parmi les polyammonium quaternaire de formule (II) utilisables selon l'invention on préfère ceux de formule (II) dans laquelle R₁, R₂, R₃ et R₄ représentent un radical méthyle ou éthyle.
   Des polymères de formule (II) particulièrement préférés sont ceux pour lesquels R₁, R₂, R₃ et R₄ représentent un radical méthyle et n = 3, p = 6 et X = Cl, et notamment ceux dont le poids moléculaire, déterminé par chromatographie par perméation de gel, est compris entre 9500 et 9900 [Polymère W].
   D'autres polymères de formule (II) particulièrement préférés sont ceux pour lesquels R₁ et R₂ représentent un radical méthyle, R₃ et R₄ représentent un radical éthyle. et n = p = 3 et X = Br, et notamment ceux dont le poids moléculaire, déterminé par chromatographie par perméation de gel, est d'environ 1200 [Polymère U]. 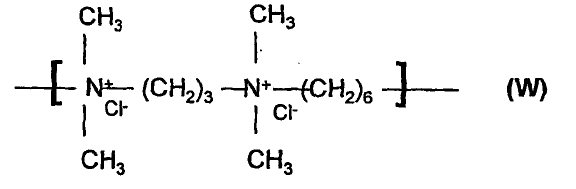 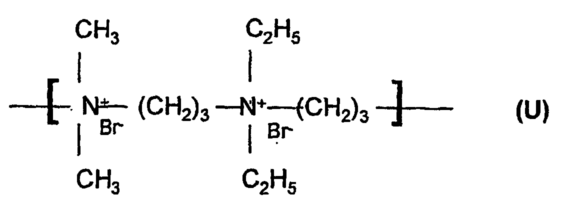 Lesdits polymères à motifs (W) et (U) sont préparés et décrits dans le brevet français 2 270 846.
**(iii)** Parmi les polymères de Vinylpyrrolidone (PVP) à motifs cationiques utilisables conformément à l'invention, on peut citer en particulier :
   a) les polymères de Vinylpyrrolidone comportant des motifs Méthacrylate de diméthylaminoéthyle ; on peut citer parmi ceux-ci :
      - le copolymère Vinylpyrrolidone / Méthacrylate de diméthylaminoéthyle (20/80 en poids) vendu sous la dénomination commerciale COPOLYMER 845 par la société I.S.P.
      - les copolymères Vinylpyrrolidone / Méthacrylate de diméthylaminoéthyle quatemisés par du sulfate de diéthyle, vendus sous les dénominations GAFQUAT 734, 755, 755 S et 755 L par la société I.S.P.
      - les PVP / Méthacrylate de diméthylaminoéthyle / Polyuréthane hydrophile, vendus sous la dénomination commerciale PECOGEL GC-310 par la société U.C.I.B. ou encore sous les dénominations AQUAMERE C 1031 et C 1511 par la société BLAGDEN CHEMICALS,
      - les PVP / Méthacrylate de diméthylaminoéthyle / Oléfine en C8 à C16, quaternisés ou non quarternisés, vendus sous les dénominations GANEX ACP 1050 à 1057, 1062 à1069, 1079 à 1086, par la société I.S.P.
      - le PVP / Méthacrylate de diméthylaminoéthyle / Vinylcaprolactame, vendu sous la dénomination GAFFIX VC 713 par la société I.S.P.
   b) les polymères de Vinylpyrrolidone comportant des motifs Méthacrylamidopropyltriméthylammonium (M.A.P.T.A.C.), parmi lesquels on peut citer notamment :
      - les copolymères Vinylpyrrolidone / M.A.P.T.A.C., vendus sous les dénominations commerciales GAFQUAT ACP 1011 et GAFQUAT HS 100 par la société I.S.P.
   c). les polymères de Vinylpyrrolidone comportant des motifs Méthylvinylimidazolium, et parmi lesquels on peut citer plus particulièrement:
      - les PVP / Chlorure de méthylvinylimidazolium, vendus sous les dénominations LUVIQUAT FC 370, FC 550, FC 905, HM 552 par la société B.A. S. F.
      - le PVP / Chlorure de méthylvinylimidazolium / Vinylimidazole, vendu sous la dénomination LWIQUAT 8155 par la société B.A.S.F.
      - le PVP / Méthosulfate de méthylvinylimidazolium, vendu sous la dénomination LUVIQUAT MS 370 par la société B.A.S.F.
**(iv)** Selon l'invention, on désigne par silicone aminée toute silicone comportant au moins une amine primaire, secondaire, tertiaire ou un groupement ammonium quaternaire .
Conformément à l'invention, les silicones aminées sont choisies parmi :
**(i)** les composés dénommés dans le dictionnaire CTFA, "amodiméthicone" et répondant à la formule (Vlll) suivante : 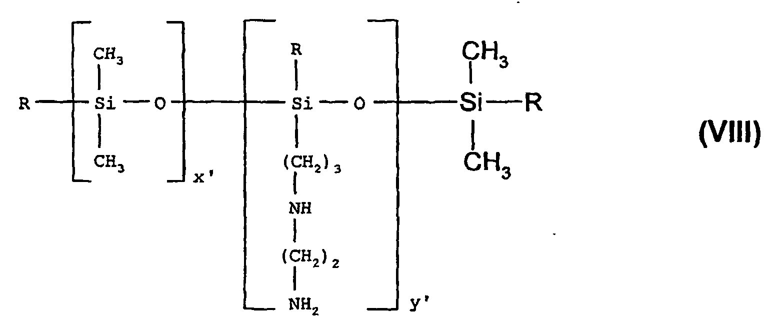 dans laquelle R désigne le radical CH₃ ou OH, et
   x' et y' sont des nombres entiers dépendant du poids moléculaire, généralement tels que ledit poids moléculaire en nombre est compris entre 5 000 et 500 000 environ;
**(ii)** les composés répondant à la formule (IX) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ **(IX)**

   dans laquelle,
   T est un atome d'hydrogène, ou un radical phényle, ou OH, ou alkyle en C₁-C₈, et de préférence méthyle,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R¹ est un radical monovalent de formule -C_{q}H_{2q}L dans laquelle q est un nombre de 2 à 8 et L est un groupement aminé éventuellement quaternisé choisi parmi les groupements :

   -N(R²)-CH₂-CH₂-N(R²)₂

   -N(R²)₂

   -N^{⊕}(R²)₃Q⁻

   -N^{⊕}(R²)(H)₂Q⁻

   -N^{⊕}(R²)₂HQ⁻

   -N(R²)-CH₂-CH₂-N^{⊕}(R²)(H)₂Q⁻,

   dans lesquels R² peut désigner hydrogène, phényle, benzyle, ou un radical hydrocarboné saturé monovalent, par exemple un radical alkyle ayant de 1 à 20 atomes de carbone et Q⁻ représente un ion halogénure tel que par exemple fluorure, chlorure, bromure ou iodure.
   Un produit correspondant à cette définition est le polymère dénommé dans le dictionnaire CTFA "triméthylsilylamodiméthicone", répondant à la formule (X) suivante 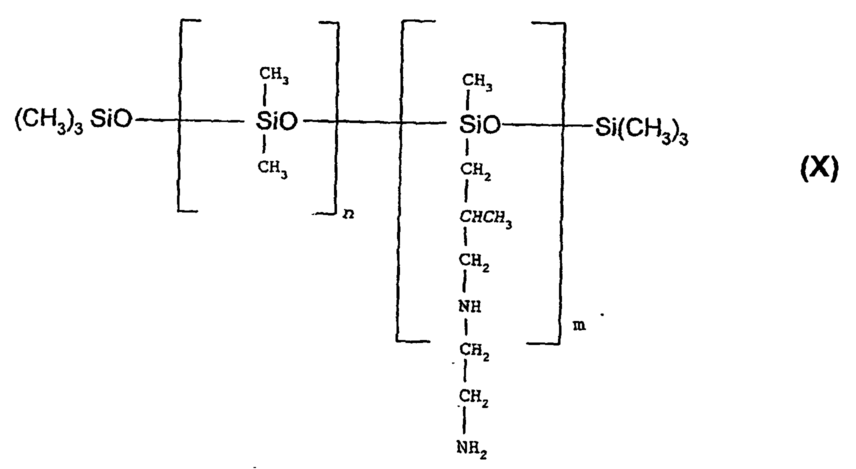 dans laquelle n et m ont les significations données ci-dessus [cf formule (IX)]. De tels composés sont décrits par exemple dans la demande de brevet EP-A-95238; un composé de formule (X) est par exemple vendu sous la dénomination Q2-8220 par la société OSI.
**(iii)** les composés répondant à la formule (XI) suivante : 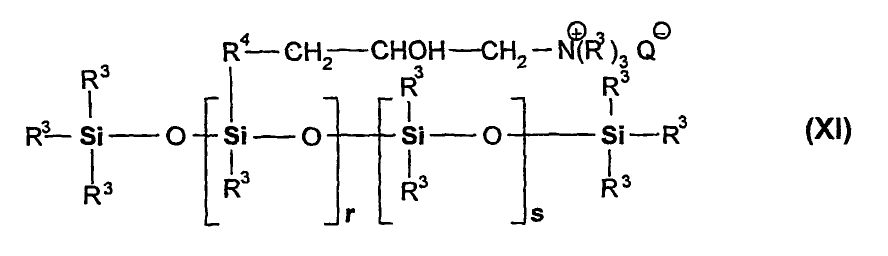 dans laquelle,
   R³ représente un radical hydrocarboné monovalent ayant de 1 à 18 atomes de carbone, et en particulier un radical alkyle en C₁-C₁₈, ou alcényle en C₂-C₁₈,
   R⁴ représente un radical hydrocarboné divalent, notamment un radical alkylène en C₁-C₁₈ ou un radical alkylèneoxy divalent en C₁-C₁₈, et par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.
   De tels composés sont décrits plus particulièrement dans le brevet US-4 185 087.
   Un composé entrant dans cette classe est celui vendu par la Société Union Carbide sous la dénomination "Ucar Silicone ALE 56".
   Lorsque ces silicones aminées sont mises en oeuvre, une forme de réalisation particulièrement intéressante est leur utilisation conjointe avec des agents de surface cationiques et/ou non ioniques.
   On peut utiliser, par exemple, le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".
   Un autre produit commercial utilisable selon l'invention est le produit vendu sous la dénomination "Dow Corning Q2 7224" par la Société Dow Corning, comportant en association le triméthylsilylamodiméthicone de formule (X) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.
**(iv)** les composés de formule (XII) suivante: 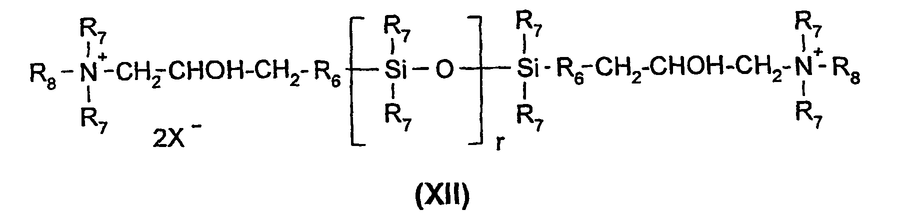 dans laquelle :
   R₆ représente un radical hydrocarboné divalent, notamment un radical alkylène en C₁-C₁₈ ou un radical alkylèneoxy divalent en C₁-C₁₈ relié au Si par une liaison SiC ;
   les radicaux R₇, identiques ou différents, représentent un radical hydrocarboné monovalent ayant de 1 à 18 atomes de carbone, un radical alcényle en C₂-C₁₈ ou un cycle comprenant 5 ou 6 atomes de carbone ;
   les radicaux R₈, identiques ou différents, représentent un atome d'hydrogène, un radical hydrocarboné monovalent ayant de 1 à 18 atomes de carbone, un radical alcényle en C₂-C₁₈ , un radical -R₆-NHCOR₇ ;
   r représente une valeur statistique moyenne de 2 à 200 ;
   X⁻ est un anion tel qu'un ion halogénure, notamment chlorure, ou un sel d'acide organique (acétate ...);
   Les silicones cationiques de formule (XII) sont par exemple décrites dans la demande EP-A-0530974.
   Des silicones entrant dans cette classe sont les silicones commercialisées par la société GOLDSCHMIDT sous les dénominations ABIL QUAT 3270, ABIL QUAT 3272, ABIL QUAT 3474.
**(v)** Parmi les homopolymères d'halogénure de diméthyldiallylammonium, on peut citer en particulier ceux du chlorure de diméthyldiallylammonium tels que celui vendu sous la dénomination "Merquat 100", par la société MERCK.
   Parmi les copolymères d'halogénure de diméthyldiallylammonium et d'acrylamide, on peut citer en particulier ceux du chlorure de diméthyldiallylammonium et d'acrylamide vendus sous les dénominations "Merquat 550" et "Merquat S", par la société MERCK.
**(vi)** Parmi les copolymères d'halogénure de diméthyldiallylammonium et d'acide acrylique, on peut citer en particulier ceux de chlorure de diméthyldiallylammonium et d'acide acrylique comme ceux proposés sous les appellations "Merquat 280", "Merquat 295" et "Merquat Plus 3330" par la société CALGON.
**(vii) et (viii)** Les copolymères d'acide (méth)acrylique, ou d'acide maléique, ou d'acide alpha-chloracrylique et de dialkylaminoalkyl(méth)acrylate ou de dialkylaminoalkyl(méth)acrylamide sont décrits dans le brevet US-3 836 537. Le copolymère acrylate de sodium / chlorure d'acrylamidopropyltriméthylammonium est vendu sous la dénomination "Polyquart KE 3033" par la société HENKEL.
**(ix)** L'Octylacrylamide/Acrylates/Butylaminoethyl Methacrylate Copolymer est une vendus sous les appellations "Amphomer" ou "Lovocryl 47" par la société dénomination CTFA (4éme Ed.., 1991) et couvre des produits tels que ceux NATIONAL STARCH.
**(x)** Les polymères comportant des motifs zwittérioniques de formule (III) peuvent également comporter des motifs dérivés de monomères non zwittérioniques tels que l'acrylate ou le méthacrylate de diméthyl- ou diéthyl-aminoéthyle ou des alkyle acrylates ou méthacrylates, des acrylamides ou méthacrylamides ou l'acétate de vinyle.
   A titre d'exemple, on peut citer le copolymère de méthacrylate de méthyle 1 diméthylcarboxyméthylammonio-éthylméthacrylate de méthyle tel que le produit vendu sous la dénomination "Diaformer Z301" par la société SANDOZ.
**(xiii)** Les copolymères alkyl(C₁-C₅)vinyléther / anhydride maléique sont modifiés partiellement par semiamidification avec une N,N-dialkylaminoalkylamine telle que la N,N-diméthylaminopropylamine ou par semiestérication avec une N,N-dialcanolamine. Ils peuvent également comporter d'autres comonomères vinyliques tels que le vinylcaprolactame.

Le ou les polymères cationiques ou amphotères ainsi définis et utilisables dans la composition tinctoriale de la présente invention peuvent représenter de 0,01 à 10% environ en poids, de préférence de 0,05 à 5% environ en poids et en particulier de 0,1 à 3% environ en poids, du poids total de la composition .

La composition de teinture selon l'invention peut également contenir un ou plusieurs coupleurs additionnels différant des 3,5-diamino-pyridines de formule (I) selon l'invention et choisis parmi les métaphénylènediamines, les métaaminophénols, les métadiphénols, les naphtols et les coupleurs hétérocycliques tels que les pyrazolo-[1,5-b]-1,2,4-triazoles, les pyrazolo-[3,2-c]-1,2,4-triazoles, les pyrazol-5-ones, les pyridines différentes des 3,5-diamino-pyridines de formule (I) selon l'invention, les indoles, les indolines, les indazoles, les benzimidazoles, les benzothiazoles, les benzoxazoles, les 1,3-benzodioxoles et les quinoünes.
Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole, le 3-(4-hydroxy-1-méthyl-1H-indol-5-ylméthyl)-1-méthyl pyridinium, et leurs sels d'addition avec un acide.

Lorsqu'ils sont présents, le ou les coupleurs additionnels représentent de préférence de 0,0001 à 10 % en poids environ du poids total de la composition tinctoriale conforme à l'invention et encore plus préférentiellement de 0,005 à 5 % en poids environ de ce poids.

Les sels d'addition avec un acide des 3,5-diamino-pyridines de formule (I), des précurseurs de colorants d'oxydation et des coupleurs additionnels éventuellement présents et utilisables dans les compositions tinctoriales selon l'invention sont notamment choisis parmi les chlorhydrates, les bromhydrates, les suifates, les tartrates, les lactates et les acétates.

La composition de teinture selon l'invention peut également contenir des colorants directs utilisés notamment pour modifier les nuances en les enrichissant de reflets; ils peuvent alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Le milieu de la composition approprié pour la teinture est de préférence un milieu aqueux constitué par de l'eau et peut avantageusement contenir des solvants organiques acceptables sur le plan cosmétique, dont plus. particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition selon l'invention peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, des conservateurs, des céramides, des pseudocéramides, des huiles végétales, minérales ou de synthèse, les vitamines ou provitamines comme le panthénol, ou des opacifiants.

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone, la 1-phényl-3-méthyl-pyrazolone, et l'acide homogentisique, et ils sont alors généralement présents dans des quantités allant d'environ 0,05 à 1,5% en poids par rapport au poids total de la composition.

La composition selon l'invention peut également contenir un ou plusieurs alcools gras, ces alcools gras étant introduits sous forme pure ou de mélange. On peut citer parmi eux plus particulièrement les alcools laurique, cétylique, stéarylique, oléique et leurs mélanges. Ces alcools gras peuvent représenter de 0,001 à 20% en poids environ du poids total de la composition.

De préférence, la composition colorante et/ou la composition oxydante de la composition prête à l'emploi selon l'invention contient au moins un tensioactif nonionique, anionique, cationique ou amphotère dans la proportion d'environ 0,1 à 20% en poids.
Encore plus préférentiellement ladite composition contient au moins un tensioactif nonionique.

Bien entendu, l'homme de l'art veillera à choisir le ou les éventuels composés complémentaires mentionnés ci-avant, de manière telle que les propriétés avantageuses attachées intrinsèquement à la composition de teinture d'oxydation selon l'invention ne soient pas, ou substantiellement pas, altérées par la ou les adjonctions envisagées.

Dans la composition oxydante, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons, le cas échéant en présence de leur donneur ou cofacteur respectif.

Le pH de la composition colorante ou de la composition prête à l'emploi et appliquée sur les fibres kératiniques [composition résultant du mélange de la composition colorante selon l'invention et de la composition oxydante], est généralement compris entre les valeurs 3 et 12. Il est de préférence compris entre 6 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium
ou de potassium et les composés de formule (XIII) suivante : 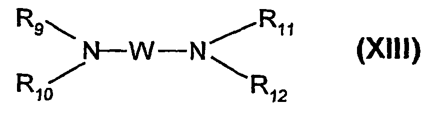 dans laquelle, W est un reste propylène éventuellement substitué par un groupement hydroxyle ou un radical alkyle en C₁-C₄ ; R₉, R₁₀, R₁₁ et R₁₂, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La composition tinctoriale conforme à l'invention peut se présenter sous des formes diverses, telles que sous forme de liquides, de poudres, de crèmes, de gels, éventuellement pressurisés, ou sous toute autre forme appropriée pour réaliser une teinture des fibres kératiniques, et notamment des cheveux humains.

Le procédé de teinture selon l'invention consiste, de préférence, à appliquer la composition prête à l'emploi, réalisée extemporanément au moment de l'emploi à partir de la composition colorante selon l'invention et de la composition oxydante décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

L'exemple qui suit est destiné à illustrer l'invention.

### EXEMPLE

On a préparé la composition tinctoriale suivante :
(exprimée en grammes - MA* désigne Matière Active)

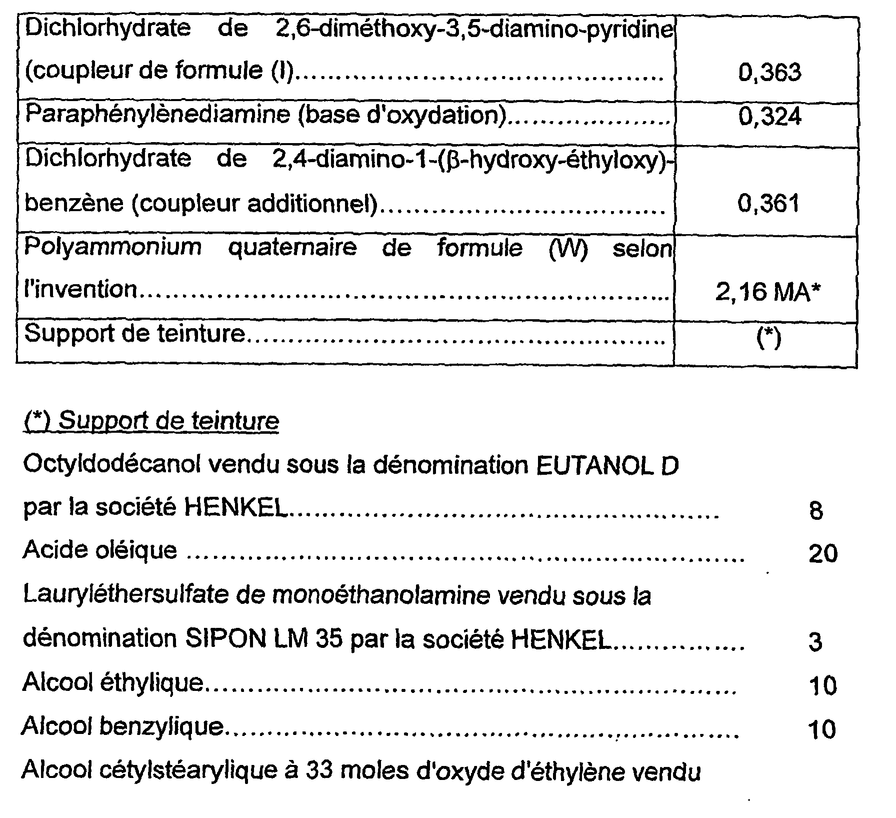

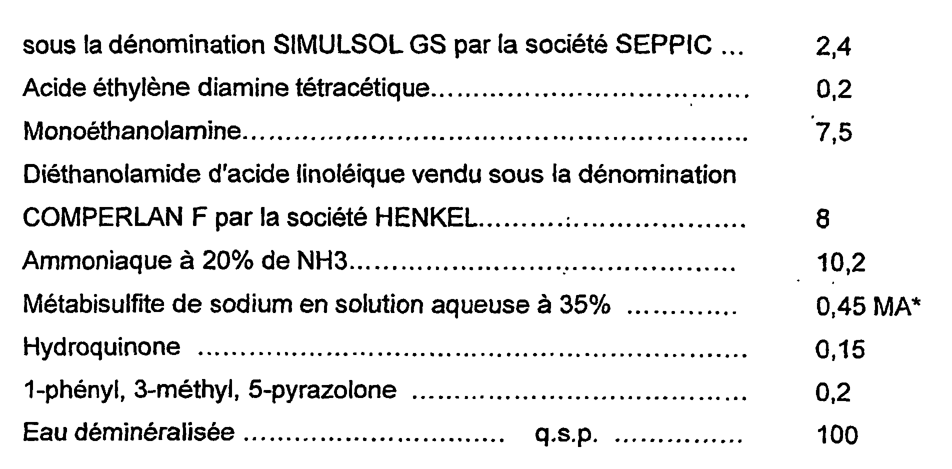

Au moment de l'emploi, on a mélangé poids pour poids la composition tinctoriale décrite ci-dessus avec une solution de peroxyde d'hydrogène à 20 volumes (6% en poids) de pH 3.

Le mélange ainsi réalisé a été appliqué pendant 30 minutes sur une mèche de cheveux gris naturels permanentés à 90 % de blancs. La mèche a ensuite été rincée, lavée avec un shampooing standard, rincée à nouveau puis séchée. Elle a été teinte dans une nuance châtain foncé très cendré.

## Revendications

1. Composition pour la teinture d'oxydation des fibres kératiniques, en particulier des fibres kératiniques humaines telles que les cheveux, comprenant, dans un milieu approprié pour la teinture,
**(a) à titre de coupleur,** au moins un dérivé de 3,5-diamino-pyridine de formule (I) suivante : 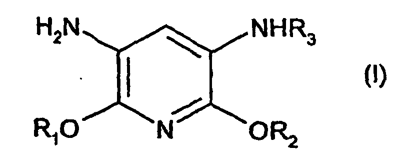 dans laquelle :
- R₁ et R₂, identiques ou différents, représentent un radical alkyle en C₁-C₄, monohydroxyalkyle en C₁-C₄, ou polyhydroxyalkyle en C₂-C₄,
- R₃ représente un atome d'hydrogène, un radical alkyle en C₁-C₄, monohydroxyalkyle en C₁-C₄ ou polyhydroxyalkyle en C₂-C₄
ou l'un de ses sels d'addition avec un acide ;
et,
(b) au moins un précurseur de colorant d'oxydation,
**caractérisée par le fait qu'**elle comprend en outre au moins un polymère cationique ou amphotère choisi dans le groupe formé par :
**(i)** les homopolymères et les copolymères d'halogénure de méthacryloyloxyéthyltriméthylammonium ;
**(ii)** les polyammonium quaternaire comportant des motifs récurrents de formules (I) ou (II) suivantes : 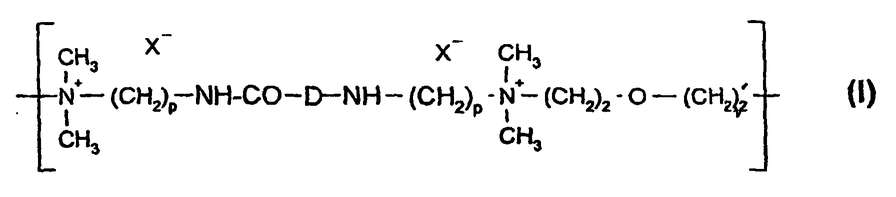 dans laquelle p désigne un nombre entier variant de 1 à 6 environ, D peut être nul ou peut représenter un groupement -(CH₂)ᵣ-CO- dans lequel r désigne un nombre égal à 4 ou à 7, X⁻ est un anion ; 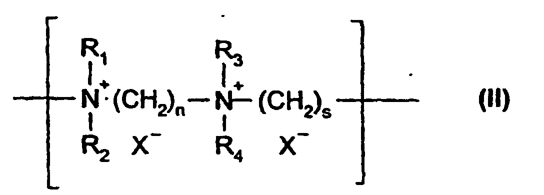 dans laquelle R₁, R₂, R₃ et R₄, identiques ou différents, désignent un radical alkyle ou hydroxyalkyle ayant de 1 à 4 atomes de carbone environ, n et s sont des nombres entiers variant de 2 à 20 et X⁻ est un anion ;
**(iii)** les copolymères de vinylpyrrolidone à motifs cationiques ;
**(iv)** les silicones aminées ;
**(v)** les homopolymères d'halogénure de diméthyldiallylammonium et les copolymères d'halogénure de diméthyldiallylammonium et d'acrylamide;
**(vi)** les copolymères d'halogénure de diméthyldiallylammonium et d'acide acrylique ;
**(vii)** les copolymères d'acide (méth)acrylique, ou d'acide maléique, ou d'acide alpha-chloracrylique et de dialkylaminoalkyl(méth)acrylate ou de dialkylaminoalkyl(méth)acrylamide ;
**(viii)** le copolymère acrylate de sodium et de chlorure d'acrylamidopropyltriméthylammonium ;
**(ix)** l'Octylacrylamide/Acrylates/Butylaminoethyl Methacrylate Copolymer ;
**(x)** les polymères comportant des motifs zwittérioniques de formule (III) suivante : 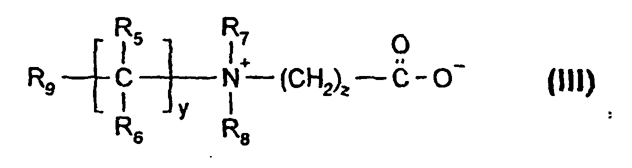 dans laquelle R₉ désigne un groupement acrylate, méthacrylate, acrylamide ou méthacrylamide, y et z représentent un nombre entier de t à 3, R₅ et R₆ représentent un atome d'hydrogène, méthyle, éthyle ou propyle, R₇ et R₈ représentent un atome d'hydrogène ou un radical alkyle de telle façon que la somme des atomes de carbone dans R₇ et R₈ ne dépasse pas 10 ;
**(xii)** Les polymères répondant à la formule générale (VII) : 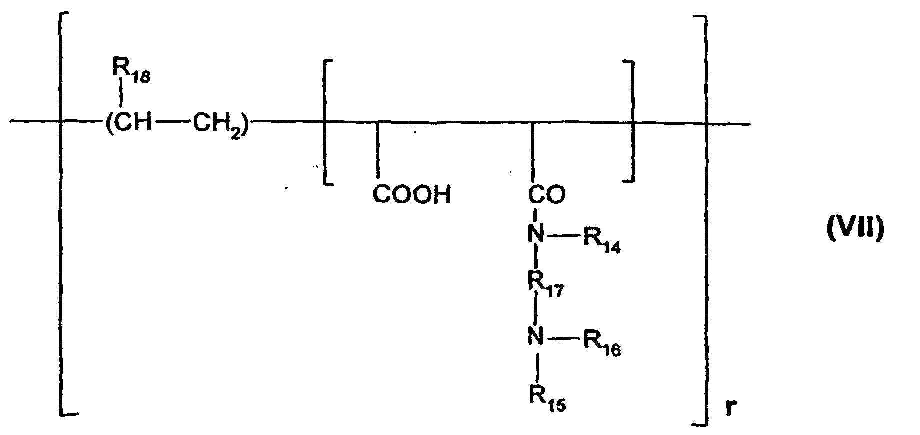 dans laquelle R₁₈ représente un atome d'hydrogène, un radical CH₃O, CH₃CH₂O, phényle, R₁₄ désigne l'hydrogène ou un radical alkyle(C₁-C₆), R₁₅ désigne l'hydrogène ou un radical alkyle(C₁-C₆), R₁₆ désigne un radical alkyle(C₁-C₆), ou un radical répondant à la formule : -R₁₇-N(R₁₅)₂, R₁₇ représentant un groupement -CH₂-CH₂-, -CH₂-CH₂-CH₂-, -CH₂-CH(CH₃)-, et R₁₅ ayant les significations mentionnées ci-dessus ;
**(xiii)** les copolymères alkyl(C₁-C₅)vinyléther / anhydride maléique modifié partiellement par semiamidification avec une N,N-dialkylaminoalkylamine ou par semiestérification avec une N,N-dialcanolamine.

2. Composition selon la revendication 1, **caractérisée par le fait que** les 3,5-diamino-pyridines de formule (I) sont choisies parmi la 2,6-diméthoxy-3,5-diamino-pyridine, la 2,6-diéthoxy-3,5-diamino-pyridine, la 2,6-di-(β-hydroxyéthyloxy)-3,5-diamino-pyridine et leurs sels d'addition avec un acide.

3. Composition selon la revendication 2, **caractérisée par le fait que** la 3,5-diamino-pyridine est la 2,6-diméthoxy-3,5-diamino-pyridine ou l'un de ses sels d'addition avec un acide.

4. Composition selon l'une quelconque des revendications précédentes, **caractérisée par le fait que** les 3,5-diamino-pyridines de formule (I) et leurs sels d'addition acide représentent de 0,0001 à 10% en poids du poids total de la composition.

5. Composition selon la revendication 4, **caractérisée par le fait que** les 3,5-diamino-pyridines de formule (I) et leurs sels d'addition acide représentent de 0,005 à 5% en poids du poids total de la composition.

6. Composition selon la revendication 1, **caractérisée par le fait que** le précurseur de colorant d'oxydation est choisi parmi les paraphénylènediamines, les bases doubles, les para-aminophénols, les ortho-aminophénols et les bases d'oxydation hétérocycliques.

7. Composition selon l'une quelconque des revendications précédentes, **caractérisée par le fait que** le ou les précurseurs de colorant d'oxydation représentent de 0,0005 à 12% en poids du poids total de la composition.

8. Composition selon la revendication 1, **caractérisée par le fait que** les polyammonium quaternaire sont choisis parmi ceux de formule (I) pour laquelle p est égal à 3, X désigne un atome de chlore et D représente un groupement -(CH₂)₄-CO-, ou un groupement -(CH₂)₇-CO-, ou la valeur zéro.

9. Composition selon la revendication 1, **caractérisée par le fait que** les polyammonium quaternaire sont choisis parmi ceux de formule (II) pour laquelle:
- ou bien R₁, R₂, R₃ et R₄ représentent un radical méthyle et n = 3, p = 6 et X = Cl, [Polymère W] ;
- ou bien R₁ et R₂ représentent un radical méthyle, R₃ et R₄ représentent un radical éthyle et n = p = 3 et X = Br, [Polymère U] :

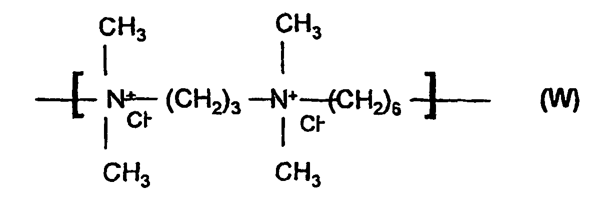

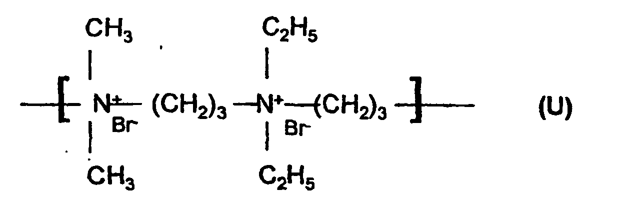

10. Composition selon la revendication 1, **caractérisée par le fait que** les polymères de vinylpyrrolidone à motifs cationiques sont choisis parmi les polymères de Vinylpyrrolidone comportant des motifs Méthacrylate de diméthylaminoéthyle , les copolymères Vinylpyrrolidone / Méthacrylate de diméthylaminoéthyle quatemisés par du sulfate de diéthyle, les Poly vinylpyrrolidone / Méthacrylate de diméthylaminoéthyle / Polyuréthane hydrophile, les Polyvinylpyrrolidone / Méthacrylate de diméthylaminoéthyle / Oléfine en C8 à C16 quatemisés ou non quartemisés, le Polyvinylpyrrolidone / Méthacrylate de diméthylaminoéthyle / Vinylcaprolactame, les polymères de Vinylpyrrolidone comportant des motifs Méthacrylamidopropyltriméthylammonium (M.A.P.T.A.C.), les polymères de Vinylpyrrolidone comportant des motifs Méthylvinylimidazolium.

11. Composition selon la revendication 1, **caractérisée par le fait que** les silicones aminées sont choisies parmi:
**(i)** l'amodiméthicone de formule (VIII) suivante : 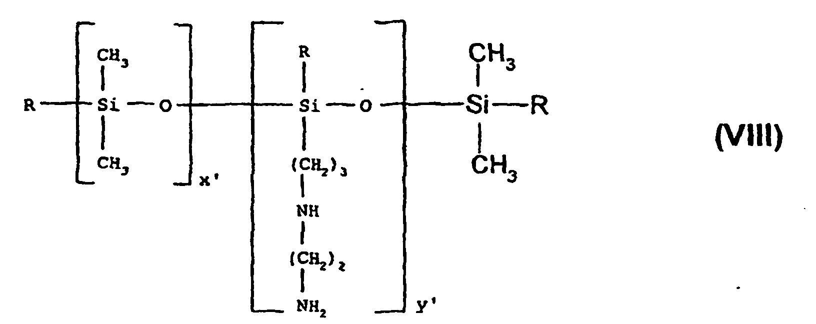 dans laquelle R désigne le radical CH₃ ou OH, et
x' et y' sont des nombres entiers dépendant du poids moléculaire, généralement tels que ledit poids moléculaire en nombre est compris entre 5 000 et 500 000 ;
**(ii)** les composés de formule (IX) suivante :
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ **(IX)**
dans laquelle,
T est un atome d'hydrogène, ou un radical phényle, ou OH, ou alkyle en C₁-C₈, et de préférence méthyle,
a désigne le nombre 0 ou un nombre entier de 1 à 3,
b désigne 0 ou 1,
m et n sont des nombres tels que la somme (n + m) peut varier notamment de 1 à 2 000, n pouvant désigner un nombre de 0 à 1 999 et m pouvant désigner un nombre de 1 à 2 000,
R¹ est un radical monovalent de formule -C_{q}H_{2q}L dans laquelle q est un nombre de 2 à 8 et L est un groupement aminé éventuellement quatemisé choisi parmi les groupements :
-N(R²)-CH₂-CH₂-N(R²)₂
-N(R²)₂
-N^{⊕}(R²)₃Q⁻
-N^{⊕}(R²)(H)₂Q⁻
-N^{⊕}(R²)₂HQ⁻
-N(R²)-CH₂-CH₂-N^{⊕}(R²)(H)₂Q⁻,
dans lesquels R² désigne hydrogène, phényle, benzyle, ou un radical alkyle ayant de 1 à 20 atomes de carbone et Q⁻ représente un ion halogénure.
**(iii)** les composés de formule (XI) suivante : 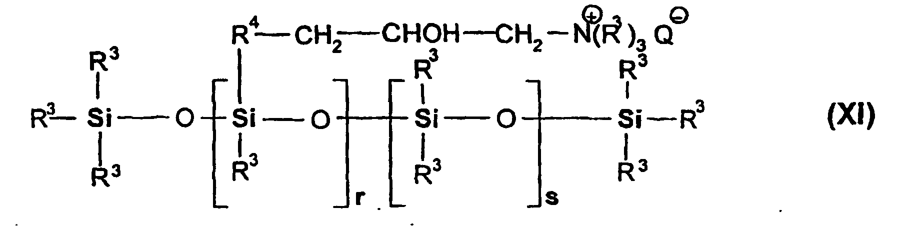 dans laquelle,
R³ représente un radical alkyle en C₁-C₁₈, ou alcényle en C₂-C₁₈,
R⁴ représente un radical alkylène en C₁-C₁₈ ou un radical alkylèneoxy divalent en C₁-C₁₈,
Q⁻ est un ion halogénure,
r représente une valeur statistique moyenne de 2 à 20,
s représente une valeur statistique moyenne de 20 à 200.
**(iv)** les composés de formule (XII) suivante: 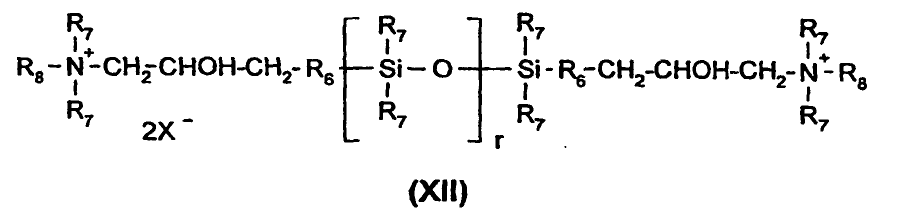 dans laquelle: .
R₆ représente un radical alkylène en C₁-C₁₈ ou un radical alkylèneoxy divalent en C₁-C₁₈ relié au Si par une liaison SiC ;
les radicaux R₇, identiques ou différents, représentent un radical hydrocarboné monovalent ayant de 1 à 18 atomes de carbone, un radical alcényle en C₂-C₁₈ ou un cycle comprenant 5 ou 6 atomes de carbone ;
les radicaux R₈, identiques ou différents, représentent un atome d'hydrogène, un radical hydrocarboné monovalent ayant de 1 à 18 atomes de carbone, un radical alcényle en C₂-C₁₈ , un radical-R₆-NHCOR₇;
r représente une valeur statistique moyenne de 2 à 200 ;
X⁻ est un anion.

12. Composition selon la revendication 11, **caractérisée par le fait que** la silicone aminée de formule (IX) est la triméthylsilylamodiméthicone.

13. Composition selon la revendication 11, **caractérisée par le fait que** l'amodiméthicone est associée au chlorure de triméthylcétylammonium et au "trideceth-12".

14. Composition selon la revendication 12, **caractérisée par le fait que** la triméthylsilylamodiméthicone est associée à l' "octoxynol-40" et à l'"isolaureth-6".

15. Composition selon l'une quelconque des revendications précédentes, **caractérisée par le fait que** le ou les polymères cationiques ou amphotères représentent de 0,01 à 10 % en poids du poids total de la composition.

16. Composition selon la revendication 15, **caractérisée par le fait que** le ou les polymères cationiques ou amphotères représentent de 0,05 à 5% en poids du poids total de la composition.

17. Composition selon la revendication 16, **caractérisée par le fait que** le ou les polymères cationiques ou amphotères représentent de 0,1 à 3% en poids du poids total de la composition.

18. Composition selon l'une quelconque des revendications précédentes, **caractérisée par le fait qu'**elle contient au moins un coupleur additionnel.

19. Composition selon la revendication 18 , **caractérisée par le fait que** les coupleurs additionnels sont choisis parmi les métaphénylènediamines, les métaaminophénols, les métadiphénols, les naphtols, les coupleurs hétérocycliques différents des 3,5-diamino-pyridines de formule (1), et les sels d'addition de ces composés avec un acide.

20. Composition selon l'une quelconque des revendications précédentes, **caractérisée par le fait que** les coupleurs sont présents dans des concentrations allant de 0,0001 à 10% en poids par rapport au poids total de la composition.

21. Composition selon l'une quelconque des revendications précédentes, **caractérisée par le fait que** les sels d'addition avec un acide des 3,5-diamino-pyridines de formule (I), des précurseurs de colorant d'oxydation et des coupleurs additionnels sont choisis parmi les chlorhydrates, les bromhydrates, les sulfates, les tartrates, les lactates et les acétates.

22. Composition selon l'une quelconque des revendications précédentes, **caractérisée par le fait qu'**elle contient en outre au moins un colorant direct dans la proportion de 0,001 à 20% en poids par rapport au poids total de la composition.

23. Composition selon l'une quelconque des revendications précédentes, **caractérisée par le fait qu'**elle contient en outre au moins un agent réducteur ou antioxydant, dans des quantités allant de 0,05 à 1,5% en poids par rapport au poids total de la composition.

24. Composition prête à l'emploi pour la teinture d'oxydation des fibres kératiniques en particulier des fibres kératiniques humaines telles que les cheveux **caractérisée par le fait qu'**elle est obtenue par mélange d'une composition colorante telle que définie à l'une quelconque des revendications 1 à 23 et d'une composition oxydante contenant au moins un agent oxydant.

25. Composition selon la revendication 24, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur ou cofacteur respectif.

26. Composition selon la revendication 25, **caractérisée par le fait que** l'agent oxydant est le peroxyde d'hydrogène.

27. Composition selon la revendication 26, **caractérisée par le fait que** l'agent oxydant est une solution d'eau oxygénée dont le titre varie de 1 à 40 volumes.

28. Composition selon l'une quelconque des revendications précédentes, **caractérisée par le fait qu'**elle possède un pH allant de 3 à 12.

29. Composition selon la revendication 24, **caractérisée par le fait que** la composition colorante et/ou la composition oxydante contient au moins un tensioactif choisi parmi les tensioactifs anioniques, cationiques, non ioniques ou amphotères dans la proportion de 0,1 à 20% en poids par rapport au poids total de la composition.

30. Procédé de teinture d'oxydation des fibres kératiniques, et en particulier des fibres kératiniques humaines telles que les cheveux, consistant à appliquer sur les fibres une composition colorante contenant, dans un milieu approprié pour la teinture, au moins une 3,5-diamino-pyridine de formule (I) telle que définie à l'une quelconque des revendications 1 à 6, la couleur étant révélée à pH alcalin, neutre ou acide à l'aide d'une composition oxydante qui est mélangée juste au moment de l'emploi à la composition colorante ou qui est appliquée séquentiellement sans rinçage intermédiaire, au moins un polymère cationique ou amphotère tel que défini à l'une quelconque des revendications 1 et 9 à 17 étant présent dans la composition colorante et/ou oxydante.

31. Dispositif à plusieurs compartiments ou " Kit " pour la teinture d'oxydation des fibres kératiniques et en particulier des fibres kératiniques humaines telles que les cheveux, **caractérisé par le fait qu'**il comporte au moins deux compartiments, dont l'un d'entre eux contient au moins une 3,5-diamino-pyridine de formule (I) telle que définie à l'une quelconque des revendications 1 à 6, et un deuxième compartiment contenant au moins un agent oxydant, au moins un polymère cationique ou amphotère tel que défini à l'une quelconque des revendications 1 et 9 à 17 étant présent dans le premier compartiment et/ou dans le second compartiment.

32. Dispositif à plusieurs compartiments ou " Kit " pour la teinture d'oxydation des fibres kératiniques et en particulier des fibres kératiniques humaines telles que les cheveux, **caractérisé par le fait qu'**il comporte au moins un compartiment contenant au moins une 3,5-diamino-pyridine de formule (I) telle que définie à l'une quelconque des revendications 1 à 6, au moins un compartiment contenant au moins un polymère cationique ou amphotère tel que défini à l'une quelconque des revendications 1 et 9 à 17, et au moins un autre compartiment contenant au moins un agent oxydant.

## Patentansprüche

1. Zusammensetzung zum oxidativen Färben von Keratinfasern und insbesondere menschlichen Keratinfasern, wie Haaren, die in einem zum Färben geeigneten Medium enthält:
(a) als Kuppler mindestens ein 3,5-Diaminopyridinderivat der folgenden Formel (I): 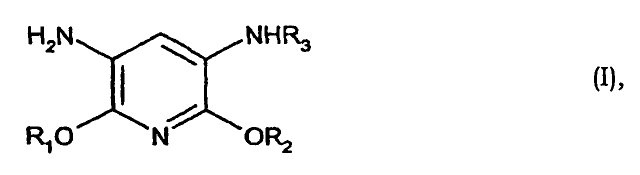 worin bedeuten:
- die Gruppen R₁ und R₂, die gleich oder verschieden sind, C₁₋₄-Alkyl, C₁₋₄-Monohydroxyalkyl oder C₂₋₄-Polyhydroxyalkyl,
- R₃ ein Wasserstoffatom, C₁₋₄-Alkyl, C₁₋₄-Monohydroxyalkyl oder C₂₋₄-Polyhydroxyalkyl; oder ein Additionssalz dieser Verbindungen mit einer Säure;
und
(b) mindestens ein Farbstoffvorprodukt eines Oxidationsfarbstoffes,
**dadurch gekennzeichnet, dass** sie ferner mindestens ein kationisches oder amphoteres Polymer enthält, das ausgewählt ist unter:
(i) Homopolymeren und Copolymeren von Methacryloyloxyethyltrimethylammoniumhalogeniden;
(ii) quartären Polyammoniumverbindungen, die wiederkehrende Einheiten der folgenden Formeln (I) oder (II) enthalten: 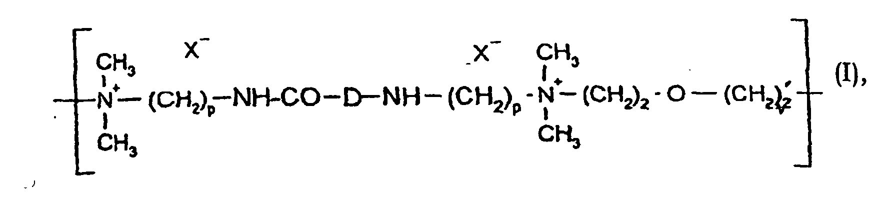 worin p eine ganze Zahl von etwa 1 bis 6 bedeutet, D nicht vorhanden sein kann oder eine Gruppe -(CH₂)ᵣ-CO- bedeutet, wobei r 4 oder 7 bedeutet, und X⁻ ein Anion ist; 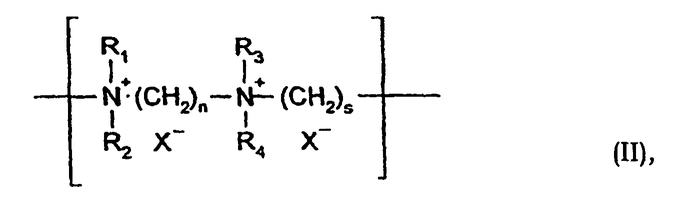 worin die Gruppen R₁, R₂, R₃ und R₄, die gleich oder verschieden sind, eine Alkyl- oder Hydroxyalkylgruppe mit etwa 1 bis 4 Kohlenstoffatomen bedeuten, n und s ganze Zahlen im Bereich von 2 bis 20 sind und X⁻ ein Anion bedeutet;
(iii) Copolymeren von Vinylpyrrolidon mit kationischen Einheiten; und
(iv) aminierten Siliconen;
(v) Homopolymeren von Dimethyldiallylammoniumhalogeniden und Copolymeren von Dimethyldiallylammoniumhalogeniden und Acrylamid;
(vi) Copolymeren von Dimethyldiallylammoniumhalogeniden und Acrylsäure;
(vii) Copolymeren von (Meth)acrylsäure oder Maleinsäure oder α-Chloracrylsäure und Dialkylaminoalkyl(meth)-acrylat oder Dialkylaminoalkyl(meth)acrylamid;
(viii) dem Copolymer von Natriumacrylat und Acrylamidopropyltrimethylammoniumchlorid;
(ix) Octylacrylamide/Acrylates/Butylaminoethyl Methacrylate Copolymer;
(x) Polymeren, die zwitterionische Einheiten der folgenden Formel (III) enthalten: 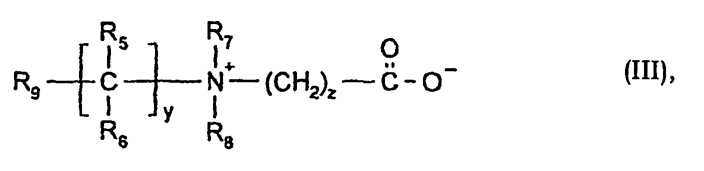 worin R₉ eine Acrylatgruppe, Methacrylatgruppe, Acrylamidgruppe oder Methacrylamidgruppe bedeutet, y und z ganze Zahlen von 1 bis 3 bedeuten, R₅ und R₆ ein Wasserstoffatom, Methyl, Ethyl oder Propyl bedeuten, R₇ und R₈ ein Wasserstoffatom oder eine Alkylgruppe bedeuten, die so gewählt ist, dass die Summe der Kohlenstoffatome in R₇ und R₈ 10 nicht übersteigt;
(xii) Polymeren der folgenden allgemeinen Formel (VII): 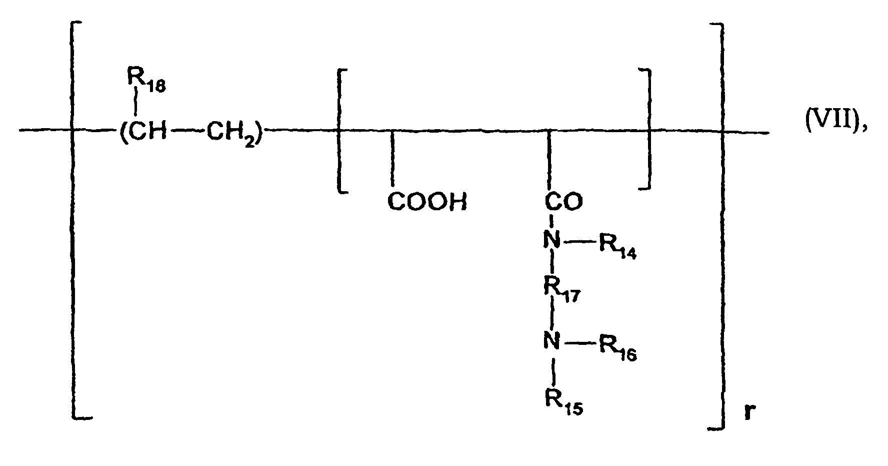 worin R₁₈ ein Wasserstoffatom, CH₃O, CH₃CH₂O oder Phenyl bedeutet, R₁₄ ein Wasserstoffatom oder eine C₁₋₆-Alkylgruppe ist, R₁₅ Wasserstoff oder C₁₋₆-Alkyl bedeutet, R₁₆ eine C₁₋₆-Alkylgruppe oder eine Gruppe der folgenden Formel ist: -R₁₇-N(R₁₅)₂, wobei R₁₇ eine Gruppe -CH₂-CH₂-, -CH₂-CH₂-CH₂- oder -CH₂CH(CH₃)- bedeutet und R₁₅ die oben angegebenen Bedeutungen aufweist;
(xiii) Copolymeren Alkyl(C₁₋₅)vinylether/Maleinsäureanhydrid, das zum Teil mit einem N,N-Dialkylamino-alkylamin semiamidiert oder einem N,N-Dialkanolamin semiverestert ist.

2. Zusammensetzung nach Anspruch 1, **dadurch gekennzeichnet, dass** die 3,5-Diaminopyridine der Formel (I) unter 2,6-Dimethoxy-3,5-diaminopyridin, 2,6-Diethoxy-3,5-diaminopyridin, 2,6-Di-(β-hydroxyethyloxy)-3,5-diaminopyridin und deren Additionssalzen mit einer Säure ausgewählt sind.

3. Zusammensetzung nach Anspruch 2, **dadurch gekennzeichnet, dass** das 3,5-Diaminopyridin das 2,6-Dimethoxy-3,5-diaminopyridin oder ein Additionssalz dieser Verbindung mit einer Säure ist.

4. Zusammensetzung nach einem der vorhergehenden Ansprüche, **dadurch gekennzeichnet, dass** die 3,5-Diaminopyridine der Formel (I) und deren Additionssalze mit einer Säure 0,0001 bis 10 Gew.-% des Gesamtgewichts der Zusammensetzung ausmachen.

5. Zusammensetzung nach Anspruch 4, **dadurch gekennzeichnet, dass** die 3,5-Diaminopyridine der Formel (I) und deren Additionssalze mit einer Säure 0,005 bis 5 Gew.-% des Gesamtgewichts der Zusammensetzung ausmachen.

6. Zusammensetzung nach Anspruch 1, **dadurch gekennzeichnet, dass** das Farbstoffvorprodukt des Oxidationsfarbstoffes unter den p-Phenylendiaminen, Doppelbasen, p-Aminophenolen, o-Aminophenolen und heterocyclischen Oxidationsbasen ausgewählt ist.

7. Zusammensetzung nach einem der vorhergehenden Ansprüche, **dadurch gekennzeichnet, dass** das oder die Farbstoffvorprodukte von Oxidationsfarbstoffen 0,0005 bis 12 Gew.-% des Gesamtgewichts der Zusammensetzung ausmachen.

8. Zusammensetzung nach Anspruch 1, **dadurch gekennzeichnet, dass** die quartären Polyammoniumverbindungen unter den Verbindungen der Formel (I), worin p 3 bedeutet, X ein Chloratom ist und D eine Gruppe -(CH₂)₄-CO- oder eine Gruppe -(CH₂)₇-CO- bedeutet oder nicht vorhanden ist, ausgewählt sind.

9. Zusammensetzung nach Anspruch 1, **dadurch gekennzeichnet, dass** die quartären Polyammoniumverbindungen unter den Verbindungen der Formel (II) ausgewählt sind, worin bedeuten:
- R₁, R₂, R₃ und R₄ Methyl und n = 3, p = 6 und X = Cl [Polymer W];
- oder R₁ und R₂ bedeuten Methyl, R₃ und R₄ bedeuten Ethyl und n = p = 3 und X = Br [Polymer U]:

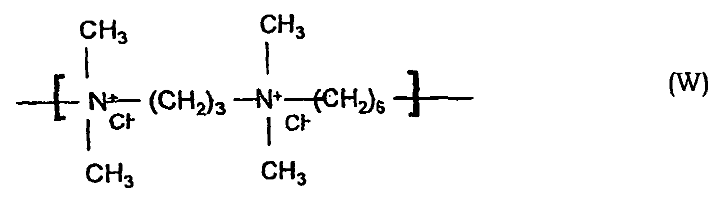

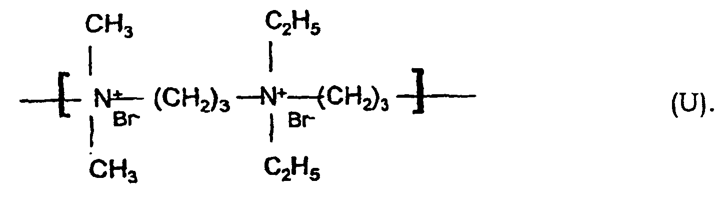

10. Zusammensetzung nach Anspruch 1, **dadurch gekennzeichnet, dass** die Vinylpyrrolidonpolymere mit kationischen Einheiten ausgewählt sind unter: Polymeren von Vinylpyrrolidon, die Dimethylaminoethylmethacrylat-Einheiten enthalten, Copolymeren Vinylpyrrolidon/Dimethylaminoethylmethacrylat, die mit Diethylsulfat quaternisiert sind, Polyvinylpyrrolidon/Dimethylaminoethylmethacrylat/hydrophiles Polyurethan-Copolymeren, Polyvinylpyrrolidon / Dimethylaminoethylmethacrylat/ C₈₋₁₆-Olefin-Copolymeren, die gegebenenfalls quaternisiert sind, dem Polyvinylpyrrolidon / Dimethylaminoethylmethacrylat/Vinylcaprolactam-Copolymer, Polymeren von Vinylpyrrolidon, die Methacrylamidopropyltrimethylammonium-Einheiten (M.A.P.T.A.C.) enthalten, Polymeren von Vinylpyrrolidon, die Methylvinylimidazolium-Einheiten enthalten.

11. Zusammensetzung nach Anspruch 1, **dadurch gekennzeichnet, dass** die aminierten Silicone ausgewählt sind unter:
(i) dem Amodimethicon der folgenden Formel (VIII): 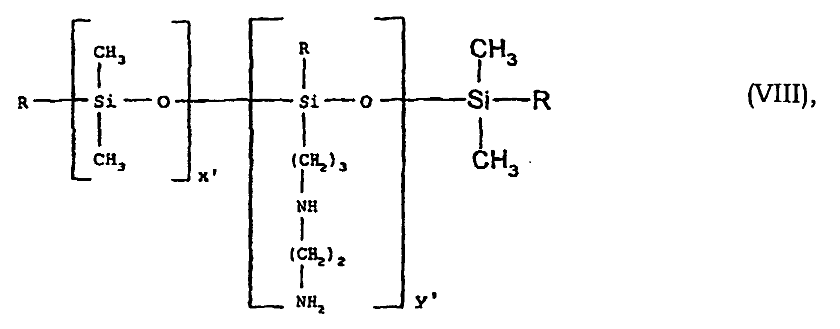 worin R die Gruppe CH₃ oder OH bedeutet, und
x' und y' ganze Zahlen sind, die von der Molmasse abhängen und im Allgemeinen so gewählt sind, dass die zahlenmittlere Molmasse im Bereich von 5 000 bis 500 000 liegt;
(ii) Verbindungen der folgenden Formel (IX):
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (IX),
worin bedeuten:
T ein Wasserstoffatom, Phenyl, OH, C₁₋₈-Alkyl und vorzugsweise Methyl,
a 0 oder eine ganze Zahl von 1 bis 3,
b 0 oder 1,
m und n ganze Zahlen, die so gewählt sind, dass die Summe (n + m) insbesondere im Bereich von 1 bis 2 000 liegen kann, wobei n eine Zahl von 0 bis 1 999 und m eine ganze Zahl von 1 bis 2 000 bedeuten kann;
R¹ eine einwertige Gruppe der Formel -C_{q}H_{2q}L, worin q eine Zahl von 2 bis 8 bedeutet und L eine gegebenenfalls quaternisierte aminierte Gruppe ist, die unter den folgenden Gruppen ausgewählt ist:
-N(R²)-CH₂-CH₂-N(R²)₂
-N(R²)₂
-N^{⊕}(R²)₃Q⁻
-N^{⊕}(R²)(H)₂Q⁻
-N^{⊕}(R²)₂HQ⁻
-N(R²)-CH₂-CH₂-N^{⊕}(R²)(H)₂Q⁻
worin die Gruppe R² Wasserstoff, Phenyl, Benzyl oder eine Alkylgruppe mit 1 bis 20 Kohlenstoffatomen bedeutet und Q⁻ ein Halogenid ist;
(iii) Verbindungen der folgenden Formel (XI): 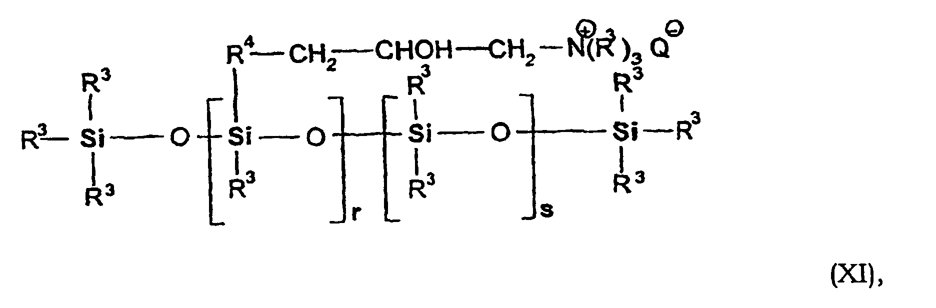 worin bedeuten:
R³ C₁₋₁₈-Alkyl oder C₂₋₁₈-Alkenyl;
R⁴ C₁₋₁₈-Alkylen oder eine zweiwertige C₁₋₁₈-Alkylenoxygruppe;
Q⁻ ein Halogenidion;
r einen statistischen Mittelwert von 2 bis 20;
s einen statistischen Mittelwert von 20 bis 200;
(iv) Verbindungen der folgenden Formel (XII): 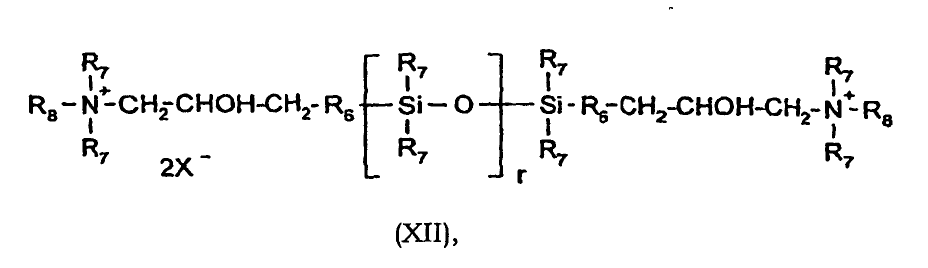 worin bedeuten:
R₆ eine C₁₋₁₈-Alkylengruppe oder eine zweiwertige C₁₋₁₈-Alkylenoxygruppe, die an das Si über eine SiC-Bindung gebunden ist;
die Gruppen R₇, die gleich oder verschieden sind, eine einwertige Kohlenwasserstoffgruppe mit 1 bis 18 Kohlenstoffatomen, C₂₋₁₈-Alkenyl oder einen Ring mit 5 oder 6 Kohlenstoffatomen;
die Gruppen R₈, die gleich oder verschieden sind, ein Wasserstoffatom, eine einwertige Kohlenwasserstoffgruppe mit 1 bis 18 Kohlenstoffatomen, C₂₋₁₈-Alkenyl oder eine Gruppe -R₆-NHCOR₇;
r einen statistischen Mittelwert von 2 bis 200;
X⁻ ein Anion.

12. Zusammensetzung nach Anspruch 11, **dadurch gekennzeichnet, dass** das aminierte Silicon der Formel (IX) das Trimethylsilylamodimethicon ist.

13. Zusammensetzung nach Anspruch 11, **dadurch gekennzeichnet, dass** das Amodimethicon mit Trimethylcetylammoniumchlorid und "Trideceth 12" kombiniert wird.

14. Zusammensetzung nach Anspruch 12, **dadurch gekennzeichnet, dass** das Trimethylsilylamodimethicon mit "Octoxynol-40" und "Isolaureth-6" kombiniert wird.

15. Zusammensetzung nach einem der vorhergehenden Ansprüche, **dadurch gekennzeichnet, dass** das oder die kationische(n) oder amphotere(n) Polymer(e) 0,01 bis 10 Gew.-% des Gesamtgewichts der Zusammensetzung ausmachen.

16. Zusammensetzung nach Anspruch 15, **dadurch gekennzeichnet, dass** das oder die kationische(n) oder amphotere(n) Polymer(e) 0,05 bis 5 Gew.-% des Gesamtgewichts der Zusammensetzung ausmachen.

17. Zusammensetzung nach Anspruch 16, , **dadurch gekennzeichnet, dass** das oder die kationische(n) oder amphotere(n) Polymer(e) 0,1 bis 3 Gew.-% des Gesamtgewichts der Zusammensetzung ausmachen.

18. Zusammensetzung nach einem der vorhergehenden Ansprüche, **dadurch gekennzeichnet, dass** sie ferner mindestens einen zusätzlichen Kuppler enthält.

19. Zusammensetzung nach Anspruch 18, **dadurch gekennzeichnet, dass** die ergänzenden Kuppler unter den m-Phenylendiaminen, m-Aminophenolen, m-Dihydroxybenzolen, Naphtholen, heterozyklischen Kupplern, die von den 3,5-Diaminopyridinen der Formel (I) verschieden sind, und den Additionssalzen dieser Verbindungen mit einer Säure ausgewählt sind.

20. Zusammensetzung nach einem der vorhergehenden Ansprüche, **dadurch gekennzeichnet, dass** die Kuppler in Konzentrationen von 0,0001 bis 10 Gew.-%, bezogen auf das Gesamtgewicht der Zusammensetzung, enthalten sind.

21. Zusammensetzung nach einem der vorhergehenden Ansprüche, **dadurch gekennzeichnet, dass** die Additionssalze der 3,5-Diaminopyridine der Formel (I), der Farbstoffvorprodukte von Oxidationsfarbstoffen und der ergänzenden Kuppler mit einer Säure unter den Hydrochloriden, Hydrobromiden, Sulfaten, Tartraten, Lactaten und Acetaten ausgewählt sind.

22. Zusammensetzung nach einem der vorhergehenden Ansprüche, **dadurch gekennzeichnet, dass** sie ferner mindestens einen Direktfarbstoff in einem Mengenanteil von 0,001 bis 20 Gew.-%, bezogen auf das Gesamtgewicht der Zusammensetzung, enthält.

23. Zusammensetzung nach einem der vorhergehenden Ansprüche, **dadurch gekennzeichnet, dass** sie ferner mindestens ein Reduktionsmittel oder Antioxidationsmittel in einem Mengenanteil von 0,05 bis 1,5 Gew.-%, bezogen auf das Gesamtgewicht der Zusammensetzung, enthält.

24. Gebrauchsfertige Zusammensetzung zum oxidativen Färben von Keratinfasern und insbesondere menschlichen Keratinfasern, wie Haaren, **dadurch gekennzeichnet, dass** sie durch Mischen einer färbenden Zusammensetzung nach einem der Ansprüche 1 bis 23 und einer oxidierenden Zusammensetzung, die mindestens ein Oxidationsmittel enthält, erhalten wird.

25. Zusammensetzung nach Anspruch 24, **dadurch gekennzeichnet, dass** das Oxidationsmittel unter Wasserstoffperoxid, Harnstoffperoxid, Alkalimetallbromaten, Alkalimetallferricyaniden, Salzen von Persäuren und Redoxenzymen, wie Laccasen, Peroxidasen und Oxidoreduktasen (2 Elektronen), gegebenenfalls in Gegenwart ihres jeweiligen Donors oder Cofaktors ausgewählt ist.

26. Zusammensetzung nach Anspruch 25, **dadurch gekennzeichnet, dass** das Oxidationsmittel Wasserstoffperoxid ist.

27. Zusammensetzung nach Anspruch 26, **dadurch gekennzeichnet, dass** das Oxidationsmittel eine Wasserstoffperoxidlösung mit einem Titer von 1 bis 40 Volumina ist.

28. Zusammensetzung nach einem der vorhergehenden Ansprüche, **dadurch gekennzeichnet, dass** sie einen pH-Wert von 3 bis 12 aufweist.

29. Zusammensetzung nach Anspruch 24, **dadurch gekennzeichnet, dass** die färbende Zusammensetzung und/oder die oxidierende Zusammensetzung mindestens einen grenzflächenaktiven Stoff, der unter den anionischen, kationischen, nichtionischen oder amphoteren grenzflächenaktiven Stoffen ausgewählt ist, in einem Mengenanteil von 0,1 bis 20 Gew.-%, bezogen auf das Gesamtgewicht der Zusammensetzung, enthält.

30. Verfahren zum oxidativen Färben von Keratinfasern, insbesondere menschlichen Keratinfasern, wie Haaren, das darin besteht, auf die Keratinfasern eine färbende Zusammensetzung, die in einem zum Färben geeigneten Medium mindestens ein 3,5-Diaminopyridin der Formel (I) nach einem der Ansprüche 1 bis 6 enthält, aufzutragen, wobei die Farbe bei einem alkalischen, neutralen oder sauren pH-Wert mit einer oxidierenden Zusammensetzung entwickelt wird, die bei der Anwendung zu der färbenden Zusammensetzung gegeben wird oder die getrennt ohne zwischenzeitliches Spülen anschließend aufgetragen wird, wobei mindestens ein kationisches oder amphoteres Polymer nach einem der Ansprüche 1 und 9 bis 17 in der färbenden Zusammensetzung und/oder der oxidierenden Zusammensetzung enthalten ist.

31. Vorrichtung mit mehreren Abteilungen oder Kit zum oxidativen Färben von Keratinfasern, insbesondere menschlichen Keratinfasern, wie Haaren, **dadurch gekennzeichnet, dass** sie mindestens zwei Abteilungen aufweist, wobei eine Abteilung mindestens ein 3,5-Diaminopyridin der Formel (I) nach einem der Ansprüche 1 bis 6 enthält und eine zweite Abteilung mindestens ein Oxidationsmittel enthält, wobei mindestens ein kationisches oder amphoteres Polymer nach einem der Ansprüche 1 und 9 bis 17 in der ersten Abteilung und/oder in der zweiten Abteilung enthalten ist.

32. Vorrichtung mit mehreren Abteilungen oder Kit zum oxidativen Färben von Keratinfasern und insbesondere menschlichen Keratinfasern, wie Haaren, **dadurch gekennzeichnet, dass** es mindestens eine Abteilung, die mindestens ein 3,5-Diaminopyridin der Formel (I) nach einem der Ansprüche 1 bis 6 enthält, mindestens eine Abteilung, die mindestens ein kationisches oder amphoteres Polymer nach einem der Ansprüche 1 und 9 bis 17 enthält und mindestens eine weitere Abteilung aufweist, die mindestens ein Oxidationsmittel enthält.

## Claims

1. Composition for the oxidation dyeing of keratin fibres, and in particular of human keratin fibres such as the hair, comprising, in a medium that is suitable for dyeing,
**(a) as coupler,** at least one 3,5-diaminopyridine derivative of formula (I) below: 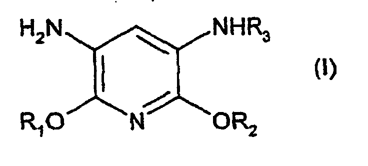 in which
- R₁ and R₂, which may be identical or different, represent a C₁-C₄ alkyl, C₁-C₄ monohydroxyalkyl or C₂-C₄ polyhydroxyalkyl radical,
- R₃ represents a hydrogen atom or a C₁-C₄ alkyl, C₁-C₄ monohydroxyalkyl or C₂-C₄ polyhydroxyalkyl radical,
or one of the addition salts thereof with an acid;
and
**(b)** at least one oxidation dye precursor,
**characterized in that** it also comprises at least one cationic or amphoteric polymer chosen from the group formed by:
**(i)** methacryloyloxyethyltrimethylammonium halide homopolymers and copolymers;
**(ii)** polyquaternary ammoniums comprising repeating units of formula (I) or (II) below: 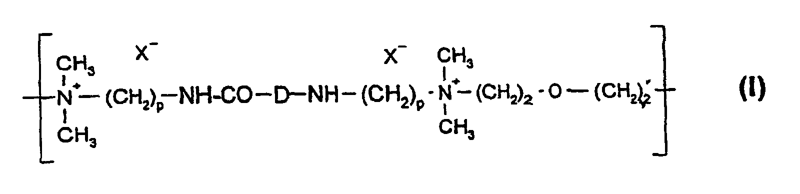 in which p denotes an integer ranging from 1 to 6 approximately, D may be zero or may represent a group -(CH₂)ᵣ-CO- in which r denotes a number equal to 4 or to 7, and X⁻ is an anion; 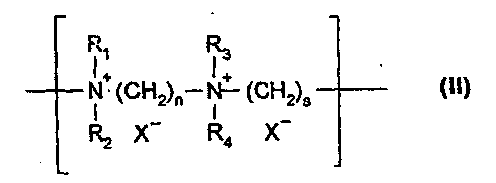 in which R₁, R₂, R₃ and R₄, which may be identical or different, denote an alkyl or hydroxyalkyl radical containing from 1 to 4 carbon atoms approximately, n and s are integers ranging from 2 to 20 and X⁻ is an anion;
**(iii)** vinylpyrrolidone copolymers containing cationic units;
**(iv)** amino silicones;
**(v)** dimethyldiallylammonium halide homopolymers and copolymers of dimethyldiallylammonium halide and of acrylamide;
**(vi)** copolymers of dimethyldiallylammonium halide and of acrylic acid;
**(vii)** copolymers of (meth)acrylic acid, or of maleic acid, or of α-chloroacrylic acid and of dialkylaminoalkyl (meth)acrylate or of dialkylaminoalkyl(meth)acrylamide;
**(viii)** the copolymer of sodium acrylate and of acrylamidopropyltrimethylammonium chloride;
**(ix)** octylacrylamide/acrylates/butylaminoethyl methacrylate copolymer;
**(x)** polymers comprising zwitterionic units of formula (III) below: 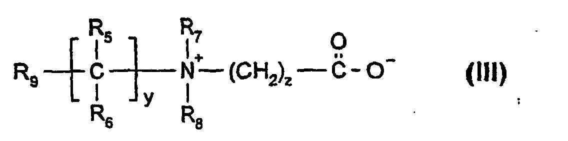 in which R₉ denotes an acrylate, methacrylate, acrylamide or methacrylamide group, y and z represent an integer from 1 to 3, R₅ and R₆ represent a hydrogen atom, methyl, ethyl or propyl, R₇ and R₈ represent a hydrogen atom or an alkyl radical such that the sum of the carbon atoms in R₇ and R₈ does not exceed 10;
**(xii)** polymers corresponding to general formula (VII): 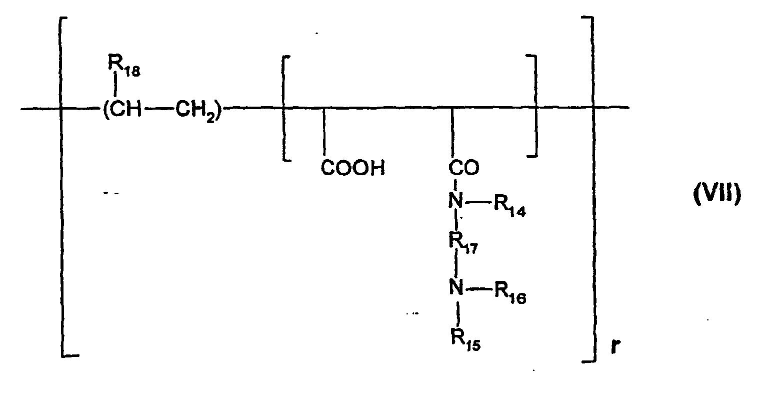 in which R₁₈ represents a hydrogen atom or a CH₃O, CH₃CH₂O or phenyl radical, R₁₄ denotes hydrogen or a (C₁-C₆)alkyl radical, R₁₅ denotes hydrogen or a (C₁-C₆)alkyl radical, R₁₆ denotes a (C₁-C₆)alkyl radical, or a radical corresponding to the formula: -R₁₇-N(R₁₅)₂, R₁₇ representing a -CH₂-CH₂-, -CH₂-CH₂-CH₂- or -CH₂-CH(CH₃)- group, and R₁₅ having the meanings given above;
**(xiii)** copolymers of (C₁-C₅)alkyl vinyl ether/maleic anhydride partially modified by semiamidation with an N,N-dialkylaminoalkylamine or by semiesterification with an N,N-dialkanolamine.

2. Composition according to Claim 1, **characterized in that** the 3,5-diaminopyridines of formula (I) are chosen from 2,6-dimethoxy-3,5-diaminopyridine, 2,6-diethoxy-3,5-diaminopyridine and 2,6-di(β-hydroxyethyloxy)-3,5-diaminopyridine, and the addition salts thereof with an acid.

3. Composition according to Claim 2, **characterized in that** the 3,5-diaminopyridine is 2,6-dimethoxy-3,5-diaminopyridine, or an addition salt thereof with an acid.

4. Composition according to any one of the preceding claims, **characterized in that** the 3,5-diaminopyridines of formula (I) and the addition salts thereof with an acid represent from 0.0001% to 10% by weight relative to the total weight of the composition.

5. Composition according to Claim 4, **characterized in that** the 3,5-diaminopyridines of formula (I) and the addition salts thereof with an acid represent from 0.005% to 5% by weight relative to the total weight of the composition.

6. Composition according to Claim 1, **characterized in that** the oxidation dye precursor is chosen from para-phenylenediamines, double bases, para-aminophenols, ortho-aminophenols and heterocyclic oxidation bases.

7. Composition according to any one of the preceding claims, **characterized in that** the oxidation dye precursor(s) represent(s) from 0.0005% to 12% by weight relative to the total weight of the composition.

8. Composition according to Claim 1, **characterized in that** the polyquaternary ammoniums are chosen from those of formula (I) for which p is equal to 3, X denotes a chlorine atom and D represents a -(CH₂)₄-CO- group or a -(CH₂)₇-CO- group, or the value zero.

9. Composition according to Claim 1, **characterized in that** the polyquaternary ammoniums are chosen from those of formula (II) for which:
- either R₁, R₂, R₃ and R₄ represent a methyl radical and n = 3, p = 6 and X = Cl [polymer W];
- or R₁ and R₂ represent a methyl radical, R₃ and R₄ represent an ethyl radical and n = p = 3 and X = Br, [polymer U]:

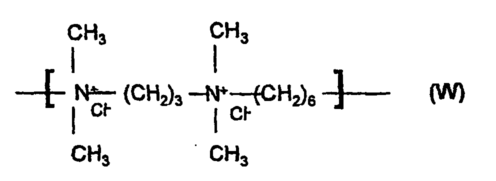

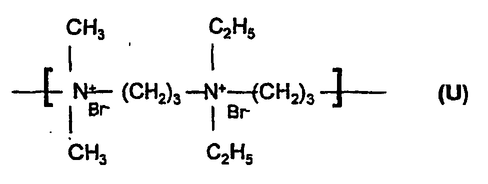

10. Composition according to Claim 1, **characterized in that** the vinylpyrrolidone polymers containing cationic units are chosen from vinylpyrrolidone polymers containing dimethylaminoethyl methacrylate units, vinylpyrrolidone/dimethylaminoethyl methacrylate copolymers quaternized with diethyl sulfate, polyvinylpyrrolidone/dimethylaminoethyl methacrylate/hydrophilic polyurethane copolymers, quaternized or nonquaternized polyvinylpyrrolidone/dimethylaminoethyl methacrylate/C8 to C16 olefin copolymers, polyvinylpyrrolidone/dimethylaminoethyl, methacrylate/vinylcaprolactam copolymers, vinylpyrrolidone polymers comprising methacrylamidopropyltrimethylammonium (MAPTAC) units, and vinylpyrrolidone polymers comprising methylvinylimidazolium units.

11. Composition according to Claim 1, **characterized in that** the amino silicones are chosen from:
**(i)** the amodimethicone of formula (VIII) below: 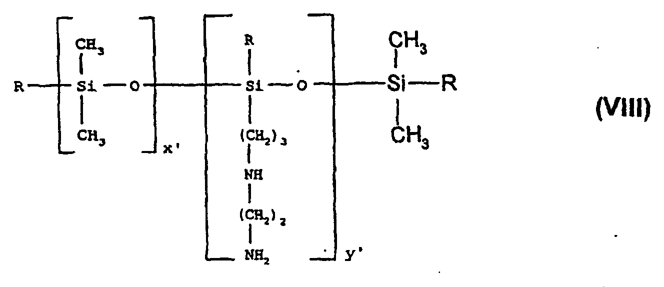 in which R denotes a CH₃ or OH radical, and
x' and y' are integers depending on the molecular weight, generally such that said number-average molecular weight is between 5000 and 500 000;
**(ii)** the compounds of formula (IX) below:
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ **(IX)**
in which
T is a hydrogen atom or a phenyl or OH or C₁-C₈ alkyl radical, and preferably methyl,
a denotes the number 0 or an integer from 1 to 3,
b denotes 0 or 1,
m and n are numbers such that the sum (n + m) can range especially from 1 to 2000, n possibly denoting a number from 0 to 1999, and m possibly denoting a number from 1 to 2000;
R¹ is a monovalent radical of formula -C_{q}H_{2q}L in which q is a number from 2 to 8 and L is an optionally quaternized amino group chosen from the following groups:
-N(R²)-CH₂-CH₂-N(R²)₂
-N(R²)₂
-N^{⊕}(R²)₃Q⁻
-N^{⊕}(R²)(H)₂Q⁻
-N^{⊕}(R²)₂HQ⁻
-N(R²)-CH₂-CH₂-N^{⊕}(R²)(H)₂Q⁻
in which R² denotes hydrogen, phenyl, benzyl or an alkyl radical containing from 1 to 20 carbon atoms, and Q⁻ represents a halide ion;
**(iii)** the compounds of formula (XI) below 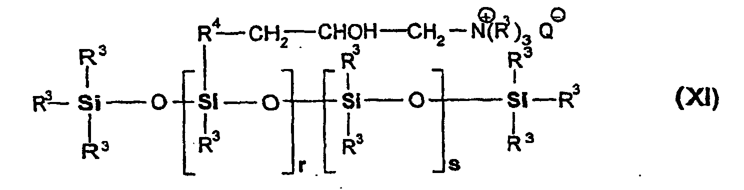 in which
R³ represents a C₁-C₁₈ alkyl or C₂-C₁₈ alkenyl radical,
R⁴ represents a C₁-C₁₈ alkylene radical or a divalent C₁-C₁₈ alkylenoxy radical,
Q⁻ is a halide ion,
r represents an average statistical value from 2 to 20,
s represents an average statistical value from 20 to 200;
**(iv)** the compounds of formula (XII) below: 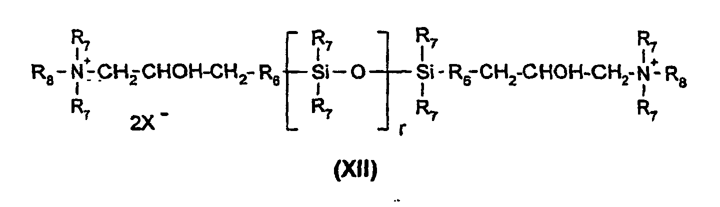 in which:
R₆ represents a C₁-C₁₈ alkylene radical or a divalent C₁-C₁₈ alkylenoxy radical linked to the Si via an SiC bond; the radicals R₇, which may be identical or different, represent a monovalent hydrocarbon radical containing from 1 to 18 carbon atoms, a C₂-C₁₈ alkenyl radical or a ring containing 5 or 6 carbon atoms;
the radicals R₈, which may be identical or different, represent a hydrogen atom, a monovalent hydrocarbon radical containing from 1 to 18 carbon atoms, a C₂-C₁₈ alkenyl radical or a radical -R₆-NHCOR₇;
r represents an average statistical value from 2 to 200;
X⁻ is an anion.

12. Composition according to Claim 11, **characterized in that** the amino silicone of formula (IX) is trimethylsilylamodimethicone.

13. Composition according to Claim 11, **characterized in that** the amodimethicone is combined with trimethylcetylammonium chloride and with "Trideceth-12".

14. Composition according to Claim 12, **characterized in that** the trimethylsilylamodimethicone is combined with "Octoxynol-40" and "Isolaureth-6".

15. Composition according to any one of the preceding claims, **characterized in that** the cationic or amphoteric polymer(s) represent(s) from 0.01% to 10% by weight relative to the total weight of the composition.

16. Composition according to Claim 15, **characterized in that** the cationic or amphoteric polymer(s) represent(s) from 0.05% to 5% by weight relative to the total weight of the composition.

17. Composition according to Claim 16, **characterized in that** the cationic or amphoteric polymer(s) represent(s) from 0.1% to 3% by weight relative to the total weight of the composition.

18. Composition according to any one of the preceding claims, **characterized in that** it contains at least one additional coupler.

19. Composition according to Claim 18, **characterized in that** the additional couplers are chosen from meta-phenylenediamines, meta-aminophenols, meta-diphenols, naphthols, heterocyclic couplers other than the 3,5-diaminopyridines of formula (I), and the addition salts of these compounds with an acid.

20. Composition according to any one of the preceding claims, **characterized in that** the couplers are present in concentrations ranging from 0.0001% to 10% by weight relative to the total weight of the composition.

21. Composition according to any one of the preceding claims, **characterized in that** the addition salts with an acid of the 3,5-diaminopyridines of formula (I), of the oxidation dye precursors and of the additional couplers are chosen from the hydrochlorides, hydrobromides, sulfates, tartrates, lactates and acetates.

22. Composition according to any one of the preceding claims, **characterized in that** it also contains at least one direct dye in a proportion of from 0.001% to 20% by weight relative to the total weight of the composition.

23. Composition according to any one of the preceding claims, **characterized in that** it also contains at least one reducing agent or antioxidant, in amounts ranging from 0.05% to 1.5% by weight relative to the total weight of the composition.

24. Ready-to-use composition for the oxidation dyeing of keratin fibres, in particular of human keratin fibres such as the hair, **characterized in that** it is obtained by mixing a dye composition as defined in any one of Claims 1 to 23 and an oxidizing composition containing at least one oxidizing agent.

25. Composition according to Claim 24, **characterized in that** the oxidizing agent is chosen from hydrogen peroxide, urea peroxide, alkali metal bromates or ferricyanides, persalts, redox enzymes such as laccases, peroxidases and 2-electron oxidoreductases, where appropriate in the presence of their respective donor or cofactor.

26. Composition according to Claim 25, **characterized in that** the oxidizing agent is hydrogen peroxide.

27. Composition according to Claim 26, **characterized in that** the oxidizing agent is an aqueous hydrogen peroxide solution with a titre ranging from 1 to 40 volumes.

28. Composition according to any one of the preceding claims, **characterized in that** it has a pH ranging from 3 to 12.

29. Composition according to Claim 24, **characterized in that** the dye composition and/or the oxidizing composition contain at least one surfactant chosen from anionic, cationic, nonionic and amphoteric surfactants in a proportion of from 0.1% to 20% by weight relative to the total weight of the composition.

30. Process for the oxidation dyeing of keratin fibres, and in particular of human keratin fibres such as the hair, which consists in applying to the fibres a dye composition containing, in a medium that is suitable for dyeing, at least one 3,5-diaminopyridine of formula (I) as defined in any one of Claims 1 to 6, the colour being developed at alkaline, neutral or acidic pH with the aid of an oxidizing composition that is mixed with the dye composition just at the time of use, or that is applied sequentially without intermediate rinsing, at least one cationic or amphoteric polymer as defined in any one of Claims 1 and 9 to 17 being present in the dye composition and/or oxidizing composition.

31. Multi-compartment device or kit for the oxidation dyeing of keratin fibres, and in particular of human keratin fibres such as the hair, **characterized in that** it comprises at least two compartments, one of which contains at least one 3,5-diaminopyridine of formula (I) as defined in any one of Claims 1 to 6, and a second compartment containing at least one oxidizing agent, at least one cationic or amphoteric polymer as defined in any one of Claims 1 and 9 to 17 being present in the first compartment and/or in the second compartment.

32. Multi-compartment device or kit for the oxidation dyeing of keratin fibres, and in particular of human keratin fibres such as the hair, **characterized in that** it comprises at least one compartment containing at least one 3,5-diaminopyridine of formula (I) as defined in any one of Claims 1 to 6, at least one compartment containing at least one cationic or amphoteric polymer as defined in any one of Claims 1 and 9 to 17, and at least one other compartment containing at least one oxidizing agent.
